# EUROPEAN PATENT APPLICATION

(11) **EP 4 331 557 A1**
(43) Date of publication of application: **06.03.2024**
(21) Application number: 22315205.9
(22) Date of filing: 01.09.2022
(51) Int. Cl.: A61K 6/20, A61K 6/61, A61K 6/887

(54) **RADICALLY POLYMERIZABLE COMPOSITION COMPRISING A REDOX INITIATOR SYSTEM BASED ON DIHYDROPYRIDINES**

(71) Applicant: Ivoclar Vivadent AG, 9494 Schaan (LI); Centre national de la recherche scientifique, 75016 Paris (FR); Université de Haute Alsace, 68093 Mulhouse Cedex (FR)
(72) Inventor: Catel, Yohann, 9475 Sevelen (CH)
(74) Representative: Uexküll & Stolberg

(57) **Abstract**

Radically polymerizable dental material, which comprises a catalyst paste and a base paste, wherein the catalyst pastes comprises an oxidizing agent, preferably a peroxide or hydroperoxide, a radically polymerizable monomer and/or oligomer with acid group and a polyfunctional radically polymerizable monomer, and the base paste comprises a polyfunctional radically polymerizable monomer without an acid group and a dihydropyridine derivative of Formula (I) 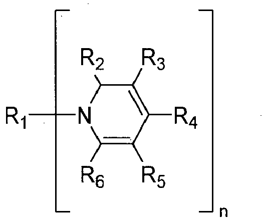 wherein R¹ is a C₆-C₁₀ aryl group, preferably a phenyl group, which can be unsubstituted or substituted; R²⁻⁶ independently of each other are a hydrogen atom, a C₁-C₂₀ alkyl group that can be interrupted by one or more of an S atom, O atom, -C(O)O- group and/or -C(O)NH-group, and/or can be unsubstituted or substituted; a C₃-C₁₆ cycloaliphatic group that can be unsubstituted or substituted; or a C₇-C₂₀-alkylaryl group that can be interrupted by one or more of an S atom, O atom, -C(O)O- group and/or -C(O)NH- group and/or can be unsubstituted or substituted; n is 1, 2, 3 or 4. The material comprises at least 50 % by weight of polyfunctional radically polymerizable monomers or monomers and oligomers, based on the total amount of radically polymerizable monomers and oligomers. The material has good mechanical properties and does not exhibit a bitter taste.

## Description

The present invention relates to radically polymerizable compositions with good mechanical properties, which do not exhibit a bitter taste and which are particularly suitable as dental materials, e.g. as dental cements, filling composites and temporary filling materials.

In the dental field, composites are mainly used for the fabrication of direct restorations or for the cementation of indirect restorations. The polymerizable organic matrix of the composites usually consists of a mixture of monomers, initiator components, pigments and stabilizers, with mixtures of dimethacrylates often being used as monomers. Such materials can be cured by thermal, redox-initiated or light-induced radical polymerization. In addition to the organic matrix, composites contain one or more fillers, which are usually surface-modified with a polymerizable coupling agent, such as 3-methacryloyloxypropyltrimethoxysilane. Fillers improve the mechanical properties (strength, modulus of elasticity, abrasion resistance) and the processing properties (paste consistency, sculptability) of the materials and impart radiopacity.

Methacrylate-based dental materials are cured by radical polymerization using radical photoinitiators, thermal initiators or redox initiator systems, depending on the field of application. Dual-curing systems contain a combination of photoinitiators and redox initiators.

Common photoinitiators are α-diketones such as e.g. camphorquinone (1,7,7-trimethyl-bicyclo[2.2.1]heptane-2,3-dione) (CQ) and 9,10-phenanthrenequinone which are often used in combination with a coinitiator.

Redox initiator systems are used in dentistry to enable the self-cure of dental materials, in particular of luting materials, composites, and temporary materials. Such initiators are key components of luting materials, which are required to mediate a bond between the tooth substance and indirect restorations, such as ceramic restorations. Luting composites and self-adhesive composite cements are among the most commonly used luting materials in dentistry. These materials are made up of an organic matrix (mixture of dimethacrylates, initiator system, additives) and inorganic fillers (silanized glass fillers, spherical mixed oxides, ytterbium fluoride, etc.). They are mostly available as light-curing (LC) or dual-curing (DC) materials. Dual-curing is essential if not enough light can reach the cement through the restoration, e.g. due to its opacity, shade or thickness. The initiator system of DC luting materials contains both a photoinitiator and a redox initiator. To ensure adequate storage stability of the redox initiators, redox initiator system-based materials are usually used as so-called 2-component systems, in which the components of the redox initiator, i.e. oxidizing agent (e.g. peroxide or hydroperoxide) and reducing agent (amines, sulfinic acids, barbiturates, thioureas, etc.), are incorporated into separate components. These are mixed together just before use. For mixing, double-push syringes are increasingly being used, which have separate cylindrical chambers for holding the components. The components are pressed out of the chambers simultaneously with two connected pistons and mixed in a nozzle. To obtain mixtures that are as homogeneous as possible, it is advantageous to mix the components in approximately equal proportions by volume.

The first paste of two-component DC cements typically contains the oxidizing agent, whereas the second paste includes a reducing agent, and often also a metal catalyst. Upon mixing of the two pastes, a redox reaction takes place, generating radicals that initiate polymerization.

Redox-initiator systems are also used in temporary dental materials as well as in DC flowable composites for the direct filling therapy. The main advantage of DC composites is their unlimited depth of cure, as light is not mandatory for the curing of the material.

Redox initiator systems based on a mixture of dibenzoyl peroxide (DBPO) with tertiary aromatic amines, such as 4-(N,N-dimethylamino)benzoic acid ethyl ester (EDMAB), N,N-diethanol-p-toluidine (DEPT), N,N-dimethyl-sym.-xylidine (DMSX) or N,N-diethyl-3,5-di-tert-butylaniline (DABA) have been widely used to initiate radically polymerization of two-component dental materials. However, this initiator system has major drawbacks. Due to the instability of DBPO, dental materials containing this peroxide must be stored in the refrigerator. In addition, the radical formation of DBPO/amine-based redox initiator systems is greatly impaired by strong acids and thus also by strongly acidic adhesive monomers which react with the amine. Moreover, oxidation of the amine can lead to discolorations of the materials. Materials comprising amines are therefore unstable.

Consequently, the dental industry intensively searched for alternative redox initiator systems that would allow the formulation of materials that can be stored at room temperature. Nowadays, hydroperoxides, such as cumene hydroperoxide (CHP) or amyl hydroperoxide, are mostly used as oxidizing agents in DC dental materials. Such hydroperoxides exhibit significantly improved stability and can be stored at room temperature. On the other hand, they do not react with amines, and therefore require alternative reducing agents.

During the past decades, reducing agents such as (thio)barbituric acid, sulfinic acid, and ascorbic acid derivatives have been evaluated in dental materials (EP 0 480 785 B1, US 6,953,535 B2, US 7,465,758 B2, EP 0 408 357 B1, US 10,932,994 B2). These reducing agents are, however, sensitive to oxygen and it is challenging to formulate storage stable materials based thereon.

For this reason, technologies based on the corresponding salts (e.g. sodium, calcium, potassium, ammonium salts) have been proposed (US 8,236,871 B2). Such salts are dispersed (insoluble) in one component of the DC cement, and an acid is added to the second component. After mixing of both components, the effective reducing agent is generated via the protonation of the corresponding salt, leading to an initiation of the polymerization.

Another redox initiator system that has been developed and used in dental materials involves (acyl)thioureas (EP 1 754 465 B1, US 10,195,121B2, US 7,541,393 B2). Redox-initiator systems based on thiourea derivatives exhibit a good acid-stability. In particular, combinations of a thiourea derivative, such as acetylthiourea, with a hydroperoxide, such as cumene hydroperoxide, are stable in the presence of strongly acidic adhesive monomers.

Redox systems comprising thiourea and hydroperoxide can be combined with transition metal compounds such as Cu(acac)₂) to improve the mechanical properties of the materials after curing; and the polymerization rate can be set by adjusting the amount of the metal compound.

Although DC dental materials based on the thiourea and hydroperoxide system are storage stable, they still have drawbacks. Thiourea derivatives have a bitter taste that can still be perceived after curing, which many patients find unpleasant.

European patent application No. 21155529.7 discloses radically polymerizable dental materials wherein the redox system comprises a hydroperoxide and a thiourea derivative comprising a radically polymerizable group. The materials can additionally contain a transition metal compound. The materials do not have a bitter taste after hardening.

EP 0 611 813 A1 discloses one-component air-activable polymerizable compositions containing a radically polymerizable compound and an activator system comprising a dihydropyridine, an onium salt and a soluble transition metal salt. The compositions do not contain a peroxides or a peroxide precursor. They are said to be stable in the absence of air and are activated by exposure to air. For bonding two substrates, at least one of the substrates is coated with the composition, exposed to oxygen for a sufficient time to activate polymerization, and then the two substrates are brought together. Such air-activatable formulations are not suitable for dental application because they are unstable in the presence of air.

EP 0 867 491 A1 discloses a two-component structural acrylic adhesive. The first component contains one or more (meth)acrylate monomer(s) or (meth)acrylated oligomers, a (meth)acrylate bearing a hydroxyl group, maleic acid, a hydroperoxide and a source of ferric ions. The second component contains a dihydropyridine derivative that may be dissolved in a (meth)acrylate monomer. The first component is characterized in that an effective amount of chelating agent is added to chelate substantially all transition metal ions that may be present before the hydroperoxide and the source of ferric ions are added. For bonding two substrates, the first component is applied to one substrate, and the second component is applied to the second substrate, and the substrates are then joined. On assembly, the two components mix and polymerization takes place.

WO 2021/150819 A1 discloses a curable composition that contains an acrylate monomer or oligomer, an alkyl peroxide and an amine. To accelerate the polymerization, 3,5-diethyl-1,2-dihydro-1-phenyl-2-propylpyridine can be added. The composition can be provided as a two-part composition, wherein the first part comprises the peroxide and an acrylic monomer, and the second part 3,5-diethyl-1,2-dihydro-1-phenyl-2-propylpyridine.

WO 2015/191189 discloses a two part adhesive composition for adhesively joining two or more pipe sections made of polyvinyl chloride, chlorinated polyvinyl chloride and/or poly(acrylonitrile-butadiene-styrene). The adhesive composition is said to be heat and moisture resistant. It comprises an initiator part comprising at least one polymer dissolved in a (meth)acrylate monomer and a free radical initiator, and an activator part comprising at least one polymer dissolved in a (meth)acrylate monomer, a pyridinic reducing agent, an organometallic curing promoter and a thiourea accelerator.

It is an object of the invention is to provide radically polymerizable materials which do not have the disadvantages of the state of the art and which are particularly suitable as dental materials. The materials are not to taste bitter, are to have a good biocompatibility and good curing characteristics even in the absence of light and to have good mechanical properties.

This object is achieved by a radically polymerizable material which comprises at least one catalyst paste and at least one base paste,
wherein the catalyst paste comprises:
   (a) at least one polyfunctional radically polymerizable monomer without an acid group,
   (b) optionally at least one monofunctional radically polymerizable monomer without an acid group,
   (c) at least one radically polymerizable monomer and/or oligomer with an acid group, and
   (d) at least one oxidizing agent;
wherein the base paste comprises:
   (e) at least one polyfunctional radically polymerizable monomer without an acid group,
   (f) optionally at least one monofunctional radically polymerizable monomer without an acid group, and
   (g) at least one dihydropyridine derivative according to Formula (I) 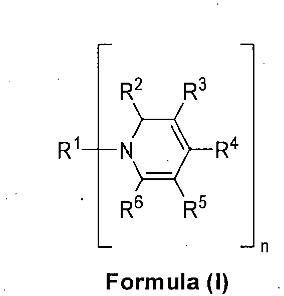 in which
      - R¹: is a C₆-C₁₀ aryl group, preferably a phenyl group, which can be unsubstituted or substituted by one or more substituents which are selected from branched or preferably linear C₁-C₂₀ alkyl groups, preferably C₁-C₁₂ alkyl groups, and more preferably C₁-C₆ alkyl groups, C₆-C₁₀ aryl groups, -O-C₁-C₁₂ alkoxy groups, -S-C₁-C₁₂ sulfide groups, CN, NO₂, ester groups -C(O)O-R⁷, where R⁷ is either a branched or preferably linear C₁-C₂₀ alkyl group, a C₆-C₁₀ aryl group or a C₇-C₂₀-alkylaryl group, and/or acyl groups -C(O)-R⁸, where R⁸ is either a branched or preferably linear C₁-C₂₀ alkyl group, a C₆-C₁₀ aryl group or a C₇-C₂₀-alkylaryl group;
      - R²⁻⁶: independently of each other are each a hydrogen atom; a branched or preferably linear C₁-C₂₀ alkyl group, preferably a C₁-C₁₂ alkyl group, and more preferably a C₁-C₆ alkyl group, that can be interrupted by one or more S atoms, O atoms, -C(O)O- groups and/or -C(O)NH- groups, and that can be unsubstituted or substituted by one or more substituents selected from C₁-C₂₀ alkyl groups, -O-C₁-C₁₂ alkoxy groups and/or ester groups -C(O)O-R⁷, where R⁷ is either a branched or preferably linear C₁-C₂₀ alkyl group, a C₆-C₁₀ aryl group or a C₇-C₂₀-alkylaryl group; a C₃-C₁₆ cycloaliphatic group that can be unsubstituted or substituted by one or more substituents selected from branched or preferably linear C₁-C₂₀ alkyl groups, preferably C₁-C₁₂ alkyl groups, most preferably C₁-C₆ alkyl groups, and that can be interrupted by one or more S atoms, O atoms, -C(O)O- groups and/or -C(O)NH- groups ; or a C₇-C₂₀-alkylaryl group that can be interrupted by one or more S atoms, O atoms, -C(O)O- groups and/or -C(O)NH- groups, and/or can be unsubstituted or substituted by one or more substituents selected from branched or preferably linear C₁-C₂₀ alkyl groups, C₆-C₁₀ aryl groups, branched or preferably linear -O-C₁-C₁₂ alkoxy groups, ester groups -C(O)O-R⁷, where R⁷ is either a branched or preferably linear C₁-C₂₀ alkyl group, a C₆-C₁₀ aryl group or a C₇-C₂₀-alkylaryl group, and/or acyl groups -C(O)-R⁸, where R⁸ is either a branched or preferably linear C₁-C₂₀ alkyl group, a C₆-C₁₀ aryl group or a C₇-C₂₀-alkylaryl group;
      - n: is 1, 2, 3 or 4, preferably 1, 2 or 3, more preferably 1 or 2,
   and wherein the material comprises at least 50 % by weight, preferably at least 60 % by weight, and more preferably at least 70 % by weight, of polyfunctional radically polymerizable monomers or monomers and oligomers, based on the total amount of radically polymerizable monomers and oligomers. More preferably the material comprises 50 % by weight to 99 % by weight, even more preferably 60 % by weight to 98 % by weight, and most preferably 70 % by weight to 97 % by weight, of polyfunctional radically polymerizable monomers or monomers and oligomers.

All formulae shown herein extend only to those compounds which are compatible with the theory of chemical valence. The indication that a group is interrupted e.g. by one or more oxygen atoms is to be understood to mean that the oxygen atoms are inserted in each case into the carbon chain of the group. The oxygen atoms are thus bordered on both sides by C atoms and cannot be terminal. C₁ radicals cannot be interrupted. Alkylaryl groups are hydrocarbon groups which contain at least one aromatic and at least one non-aromatic residue, for example a -CH₂-CH₂-Ph or a -CH₂-Ph group with Ph = penyl.

Groups such as -C(O)O- group and -C(O)NH- can be arranged in any desired orientation. For example, -C(O)O- represents both -C(O)O- and -OC(O)-.

Preferably the variables of Formula (I) have the following meanings:
- R¹: is a C₆-C₁₀ aryl group, preferably a phenyl group, which can be unsubstituted or substituted by one or more, preferably 1 to 3, more preferably 1 or 2, and most preferably 1 substituent which is/are preferably selected from C₆-C₁₀ aryl groups, -O-C₁-C₁₂ alkoxy groups, -O-C₁-C₁₂ sulfide groups, CN, NO₂, ester groups -C(O)O-R⁷, where R⁷ is a branched or preferably linear C₁-C₂₀ alkyl group, and/or acyl groups -C(O)-R⁸, where R⁸ is a branched or preferably linear C₁-C₂₀ alkyl group;
- R⁴, R⁶: are each a hydrogen atom;
- R², R³, R⁵: independently of each other are each a branched or preferably linear C₁-C₂₀, preferably C₁-C₁₆, more preferably C₁-C₁₀, and most preferably C₁-C₆ alkyl group that can be interrupted by one or more S atoms, O atoms, -C(O)O- groups and/or -C(O)NH- groups and is preferably not interrupted, a C₃-C₁₆ cycloaliphatic group that is preferably unsubstituted or is substituted by one or more, preferably 1 to 3, more preferably 1 or 2, and most preferably 1 branched or preferably linear C₁-C₁₀ alkyl group that can be interrupted by one or more, preferably 1 to 3, more preferably 1 or 2 and even more preferably 1 S atom, O atom, -C(O)O-group and/or -C(O)NH- group and is most preferably not interrupted; or a C₇-C₂₀-alkylaryl group that can be interrupted by one or more, preferably 1 to 3, more preferably 1 or 2, and even more preferably 1 S atom, O atom, -C(O)O- group and/or -C(O)NH- group and is most preferably not interrupted, and/or can be substituted by one or more, preferably 1 to 3, more preferably 1 or 2, and even more preferably 1 substituent selected from branched or preferably linear C₁-C₂₀ alkyl groups, C₆-C₁₀ aryl groups, -O-C₁-C₁₂ alkoxy groups, ester groups -C(O)O-R⁷, where R⁷ is either a branched or preferably linear C₁-C₂₀ alkyl group, a C₆-C₁₀ aryl group or a C₇-C₂₀-alkylaryl group, and/or acyl groups -C(O)-R⁸, where R⁸ is either a branched or preferably linear C₁-C₂₀ alkyl group, a C₆-C₁₀ aryl group or a C₇-C₂₀-alkylaryl group, and is most preferably not substituted,
- n: 1.

More preferably the variables of Formula (I) have the following meanings:
- R¹: is a phenyl group, which is unsubstituted or substituted by one substituent selected from a phenyl group, linear -O-C₁-C₄ alkoxy groups, preferably -OCH₃, -O-C₁-C₄ sulfide groups, preferably -SCH₃, CN, NO₂, ester groups -C(O)O-R⁷, where R⁷ is a branched or preferably linear C₁-C₄ alkyl group, preferably methyl or ethyl;
- R⁴, R⁶: are each a hydrogen atom;
- R², R³, R⁵: independently of each other are each H, preferably a C₇-C₁₀-alkylaryl group, especially -CH₂-Ph or -CH₂CH₂-Ph, more preferably a linear C₁-C₆ alkyl group that can be substituted by one -C(O)O-R⁷ group, where R⁷ is ethyl or preferably methyl, or is preferably unsubstituted,
- n: 1.

According to one embodiment of the present invention, R¹ is a C₆-C₁₀ aryl group, preferably a phenyl group, which is substituted by an electron withdrawing group, preferably in the *para* position. An electron withdrawing group is a group that reduces the electron density in a molecule. Exemplary electron withdrawing groups are nitro groups, aldehyde groups, ketone groups, cyano groups, carboxylic acid groups, ester groups and halogens (-F, -CI, -Br, -I). Preferred electron withdrawing groups are halogen atoms, in particular Br or Cl, more preferably ester groups -C(O)O-R⁷, where R⁷ is a branched or preferably linear C₁-C₁₀ alkyl group, acyl groups -C(O)-R⁸, where R⁸ is a branched or preferably linear C₁-C₁₀ alkyl group, and most preferably CN or NO₂. R² to R⁶ and n have the meanings, preferred meanings and more preferred meanings defined above. It was surprisingly found that dihydropyridine derivatives according to Formula I wherein R¹ is a C₆-C₁₀ aryl group, preferably a phenyl group, which is substituted by an electron withdrawing group have improved storage stability in the presence or radically polymerizable monomers and are more stable in the presence of air. Without wishing to be bound by any theory, it is assumed that improved stability is due to the presence of the electron-withdrawing group on the phenyl ring of R¹.

Particularly preferred compounds according to Formula (I) comprising an electron withdrawing group are compounds wherein the variables of Formula (I) have the following meanings:
- R¹: is a phenyl group, which is substituted by one or more, preferably 1 to 3, more preferably 1 to 2, and most preferably 1 electron withdrawing substituent which is/are preferably selected from ester groups -C(O)O-R⁷, where R⁷ is a branched or preferably linear C₁-C₁₀ alkyl group, acyl groups -C(O)-R⁸, where R⁸ is a branched or preferably linear C₁-C₁₀ alkyl group, and more preferably CN and/or NO₂;
- R⁴, R⁶: are each a hydrogen atom;
- R², R³, R⁵: independently of each other are each a branched or preferably linear C₁-C₆ alkyl group that can be interrupted by one or more, preferably 1 to 3, more preferably 1 or 2, and even more preferably 1 S atom, O atom, -C(O)O- group and/or -C(O)NH-group and which is most preferably not interrupted,
- n: 1.

Most preferably the variables of Formula (I) have the following meanings:
- R¹: is a phenyl group, which is substituted by a electron withdrawing substituent in the *para* position which is preferably selected from ester groups -C(O)O-R⁷, where R⁷ is a branched or preferably linear C₁-C₆ alkyl group, acyl groups -C(O)-R⁸, where R⁸ is a branched or preferably linear C₁-C₆ alkyl group, CN or NO₂;
- R4, R⁶: are each a hydrogen atom;
- R², R³, R⁵: independently of each other are each a linear C₁-C₆ alkyl group
- n: 1.

According to another embodiment of the present invention, R¹ is a C₆-C₁₀ aryl group, preferably a phenyl group, which is substituted by an electron donating group, preferably in the *para* position. An electron donating group is a group that increases the electron density in a molecule. Exemplary electron donating groups are amino groups, alkoxy groups, sulfide groups and alkyl groups. Preferred electron donating groups are branched or preferably linear C₁-C₂₀ alkyl groups, more preferably branched or preferably linear -O-C₁-C₁₂ alkoxy groups or branched or preferably linear -S-C₁-C₁₂ sulfide groups, and most preferably a branched or preferably linear -O-C₁-C₄ alkoxy groups. R² to R⁶ and n have the meanings, preferred meanings and more preferred meanings defined above. It was surprisingly found that dihydropyridine derivatives according to Formula I wherein R¹ is a C₆-C₁₀ aryl group, preferably a phenyl group, which is substituted by an electron donating group, initiate polymerization particularly well in combination with an oxidizing agent.

Particularly preferred compounds according to Formula (I) comprising an electron donating group are compounds wherein the variables of Formula (I) have the following meanings:
- R¹: is a phenyl group, which is substituted by one or more, preferably 1 to 3, more preferably 1 to 2, and most preferably 1 electron donating substituent which is/are preferably selected from branched or preferably linear C₁-C₂₀ alkyl groups, more preferably branched or preferably linear -O-C₁-C₁₂ alkoxy groups or branched or preferably linear -S-C₁-C₁₂ sulfide groups, and most preferably branched or preferably linear -O-C₁-C₄ alkoxy groups;
- R⁴, R⁶: are each a hydrogen atom;
- R², R³, R⁵: independently of each other are each a branched or preferably linear C₁-C₆ alkyl group that can be interrupted by one or more, preferably 1 to 3, more preferably 1 or 2, and even more preferably 1 S atom, O atom, -C(O)O- group and/or -C(O)NH-group and is most preferably not interrupted,
- n: 1.

Most preferably the variables of Formula (I) have the following meanings:
- R¹: is a phenyl group, which is substituted by a electron donating substituent in the *para* position which is selected from branched or preferably linear -O-C₁-C₁₀ alkoxy groups, preferably branched or preferably linear -O-C₁-C₄ alkoxy groups;
- R⁴, R⁶: are each a hydrogen atom;
- R², R³, R⁵: independently of each other are each a branched or preferably linear C₁-C₆ alkyl group
- n: 1.

The radically polymerizable compositions according to the invention can also comprise at least one dihydropyridine derivative according to Formula (I) wherein R¹ is a C₆-C₁₀ aryl group, preferably a phenyl group, which is substituted by an electron withdrawing group, and at least one dihydropyridine derivative according to Formula (I) wherein R¹ is a C₆-C₁₀ aryl group, preferably a phenyl group, which is substituted by an electron donating group, as defined above.

The preferred, more preferred and most preferred definitions given for the individual variables can be selected in each case independently of each other. Compounds in which all the variables have the preferred, more preferred and most preferred definitions are naturally particularly suitable according to the invention.

Dihydropyridine derivatives according to Formula (I), wherein R¹ is a C₆-C₁₀ aryl group, preferably a phenyl group, which is substituted by one or more substituents, which are preferably selected from branched or preferably linear C₁-C₂₀ alkyl groups, C₆-C₁₀ aryl groups, -O-C₁-C₁₂ alkoxy groups, -S-C₁-C₁₂ sulfide groups, CN, NO₂, ester groups -C(O)OR⁷, where R⁷ is either a branched or preferably linear C₁-C₂₀ alkyl group, a C₆-C₁₀ aryl group or a C₇-C₂₀-alkylaryl group, and/or acyl groups -C(O)-R⁸, where R⁸ is either a branched or preferably linear C₁-C₂₀ alkyl group, a C₆-C₁₀ aryl group or a C₇-C₂₀-alkylaryl group, and wherein R² to R⁶ and n have the meanings defined above are also subject of the present invention. Particularly preferred are the dihydropyridine derivatives with an electron withdrawing group or an electron donating group as defined above.

Dihydropyridines of Formula (I) can be prepared according to known synthetic pathways. As an example, they can be prepared via the reaction of an aromatic amine with an aldehyde in acidic aqueous solution, e.g., DHP1 (CAS Registry Number 34562-31-7) can be obtained as follows:

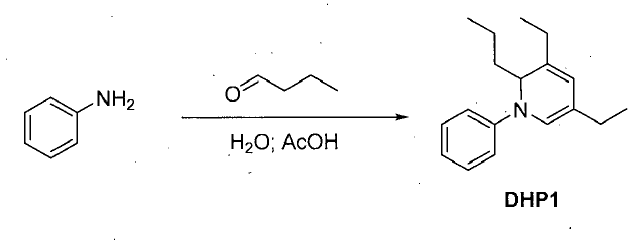

Dihydropyridines with electron withdrawing substituents Y on the aromatic ring, such as nitro, nitrile, or ester groups, can be synthesized by palladium catalyzed ipso substitution of corresponding aryl halides: 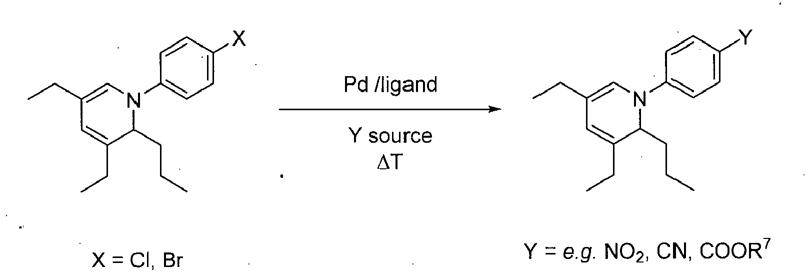
Y is preferably NO₂, CN, or C(O)OR⁷, wherein R⁷ is defined as above and is preferably ethyl. The Y source is preferably NaNO₂, K₄[Fe(CN)₆], or diethyl oxalate, respectively.
X is a leaving group, preferably a halogen atom and more preferably Cl, Br or I.
Preferred palladium catalysts (Pd/ligand) are tris(dibenzylideneacetone)dipalladium (Pd₂(bda)₃) and Pd(OAc)₂.

Particularly preferred dihydropyridines according to the Formula (I) are

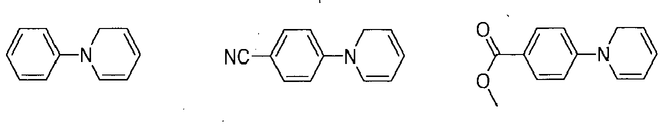

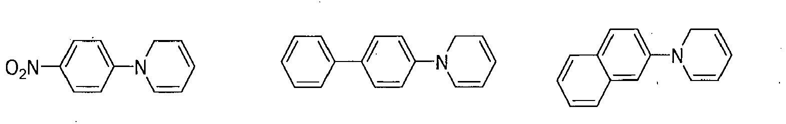

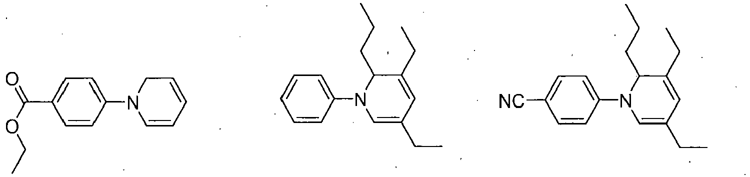

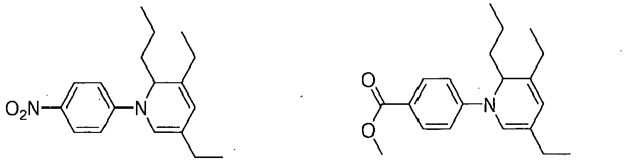

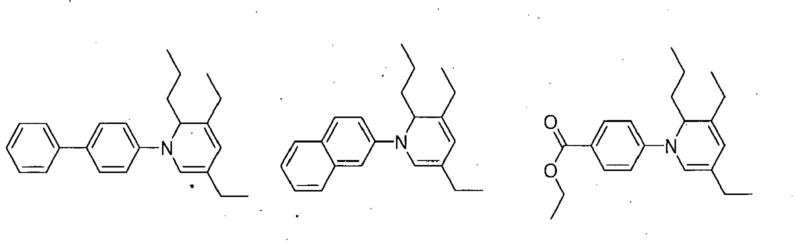

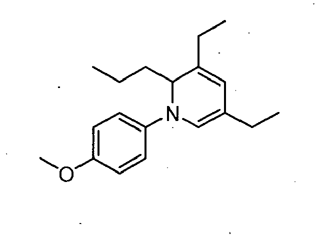

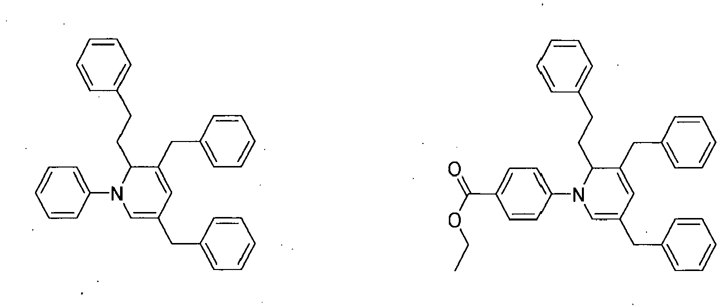

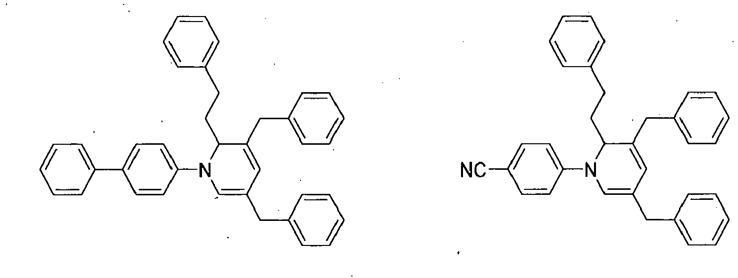

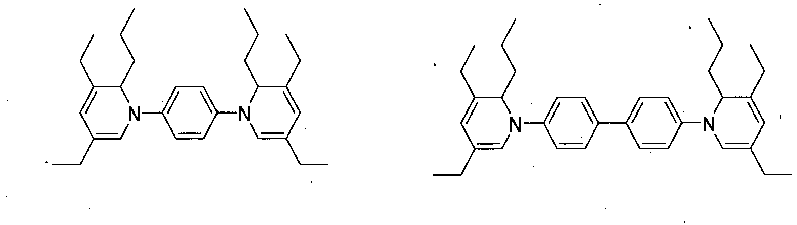

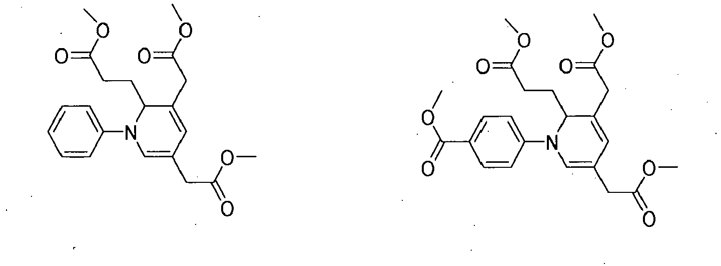

The materials according to the present invention contain at least one **oxidizing agent** in the catalyst paste. Preferred oxidizing agents are peroxides and in particular hydroperoxides. A particularly preferred peroxide is tert-butyl peroxybenzoate.

Preferred hydroperoxides are compounds of the formula R⁹-(OOH)ₘ, in which R⁹ is an aliphatic or aromatic hydrocarbon radical and m is 1 or 2. Preferred radicals R⁹ are alkyl and aryl groups. The alkyl groups can be straight-chain, branched or cyclic. Cyclic alkyl radicals can be substituted by aliphatic alkyl groups. Alkyl groups with 4 to 10 carbon atoms are preferred. Aryl groups can be unsubstituted or substituted by alkyl groups. Preferred aromatic hydrocarbon radicals are benzene radicals which are substituted with 1 or 2 alkyl groups. The aromatic hydrocarbon radicals preferably contain 6 to 12 carbon atoms.

Particularly preferred hydroperoxides are t-amyl hydroperoxide, 1,1,3,3-tetramethylbutyl hydroperoxide, t-butyl hydroperoxide, t-hexyl hydroperoxide, 2,5-dimethyl-2,5-di(hydro-peroxy)hexane, diisopropylbenzene monohydroperoxide, paramenthane hydroperoxide, p-isopropylcumene hydroperoxide and mixtures thereof. Cumene hydroperoxide (CHP) is quite particularly preferred.

Further preferred are the low-odor CHP derivatives of Formula II disclosed in European patent application EP 3 692 976 A1: 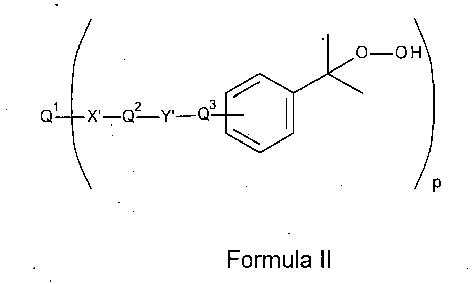 in which the variables have the following meanings:
- Q¹: a p-valent, aromatic, aliphatic, linear or branched C₁-C₁₄ hydrocarbon radical, which can be interrupted by one or more S and/or O atoms and which can be unsubstituted or substituted by one or more substituents which are preferably selected from -OH, -OR¹⁰, -Cl and -Br, wherein R¹⁰ is an aliphatic, linear or branched C₁-C₁₀ hydrocarbon radical,
- X', Y': independently of each other are in each case absent, -O-, -COO-; -CONR¹¹-, or -O-CO-NR¹²-, wherein R¹¹ and R¹² independently of each other represent H or a C₁- C₅ alkyl radical, preferably H, methyl and/or ethyl, particularly preferably H, and wherein X' and Y' are preferably not absent at the same time and wherein X' and/or Y' is absent if Q² is absent,
- Q²: is absent, an aliphatic, linear or branched C₁-C₁₄ alkylene radical, which can be interrupted by S and/or O atoms and which can be unsubstituted or substituted by -OH, -OR¹³, -Cl and/or -Br, wherein R¹³ is an aliphatic, linear or branched C₁-C₁₀ hydrocarbon radical,
- Q³: a C₁-C₃ alkylene group or is absent, preferably -CH₂- or is absent,
- p: 1, 2, 3 or 4,
and wherein the substitution on the aromatic compound takes place in position 2, 3 or 4, relative to the cumene hydroperoxide group.

The variables of Formula (II) preferably have the following meanings: .
- Q¹: a mono- or divalent, aliphatic, linear or branched C₁-C₁₀ hydrocarbon radical, which can be interrupted by one or more O atoms, preferably one O atom, and which can be substituted by one or more, preferably one, substituents which are selected from -OH and -OR¹⁰ or is preferably unsubstituted, wherein R¹⁰ is an aliphatic, linear or branched C₁-C₆ hydrocarbon radical,
- X', Y': independently of each other are in each case absent, -O-, -COO- or -O-CO-NR¹²-, wherein R¹² represents H or a C₁-C₅ alkyl radical, preferably H, methyl and/or ethyl and quite particularly preferably H, and wherein X' and Y' are preferably not absent at the same time,
- Q²: is absent, a linear or branched C₁-C₁₀ alkylene radical, which can be interrupted by one or more O atoms, preferably one O atom, and which can be substituted by one or more, preferably one, substituents which are selected from -OH and - OR¹³ or is preferably unsubstituted, wherein R¹³ is an aliphatic, linear or branched C₁-C₆ hydrocarbon radical,
- p: 1 or 2,
and wherein the substitution on the aromatic compound takes place in position 3, preferably in position 4.

More preferably the variables of Formula (II) have the following meanings:
- Q¹: a mono- or divalent, aliphatic, linear or branched C₁-C₅ hydrocarbon radical, which can be interrupted by one O atom and which can be substituted by one OH group,
- X': -COO-,
- Y': is absent,
- Q²: is absent or a linear C₁-C₃ alkylene radical,
- p: 1 or 2,
and wherein the substitution on the aromatic compound takes place in position 4.

Most preferably the variables of Formula (II) have the following meanings:
- Q¹: a mono- or divalent, aliphatic, branched, preferably linear C₁-C₄ hydrocarbon radical,
- X: -COO-,
- Y: is absent,
- Q²: is absent with or a methylene radical,
- p: 1 or 2, and wherein the substitution on the aromatic compound takes place in position 4.

Hydroperoxide derivatives of Formula II preferred according to the invention are:

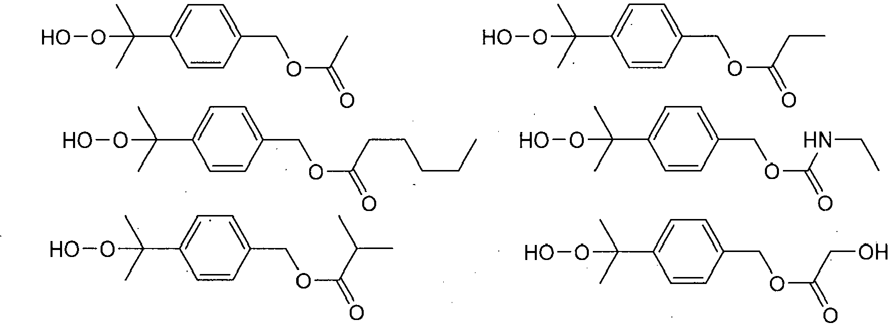

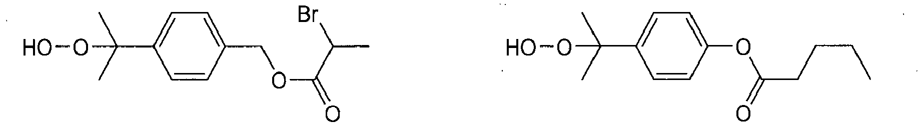

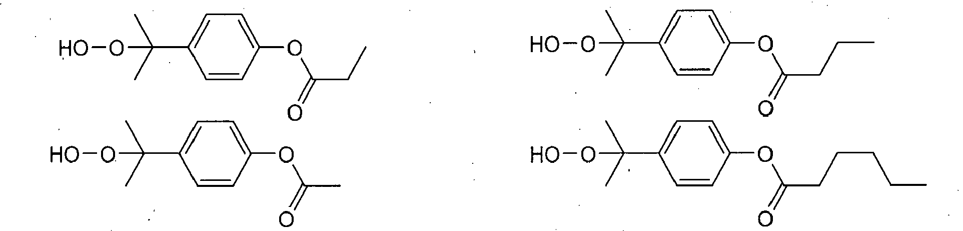

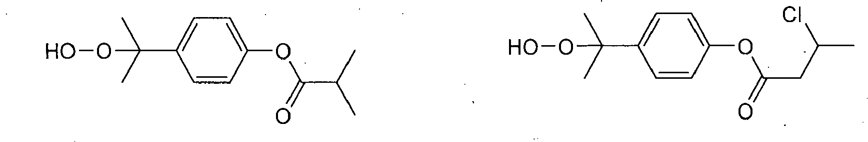

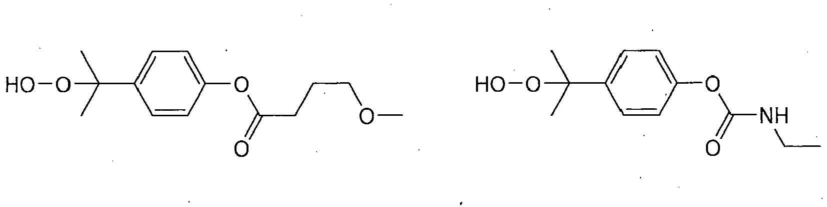

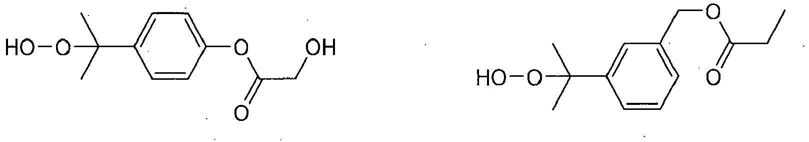

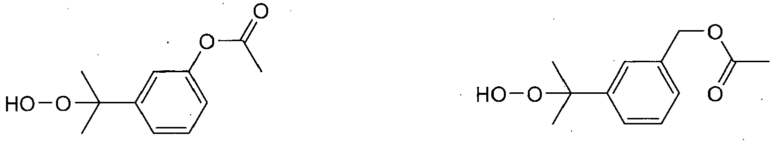

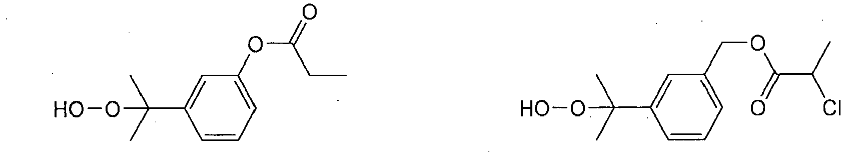

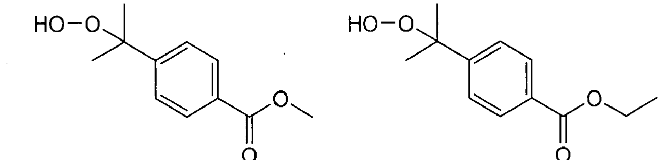

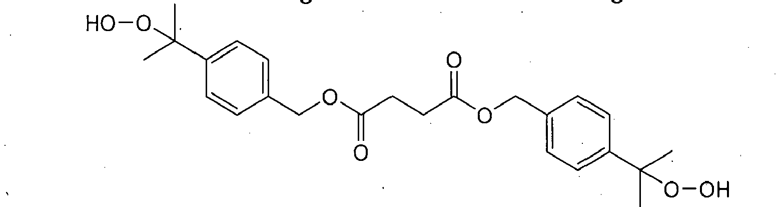

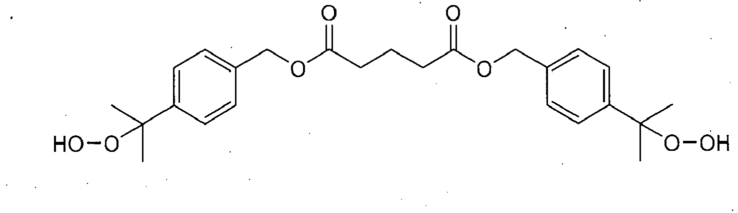

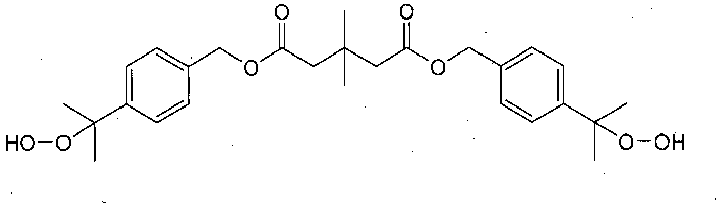

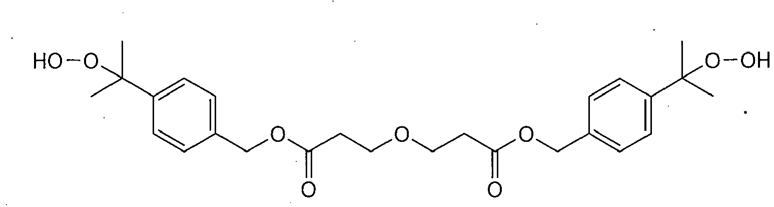

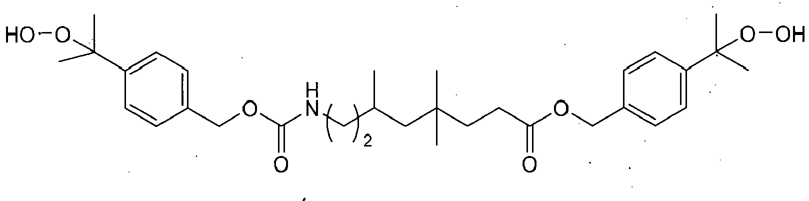

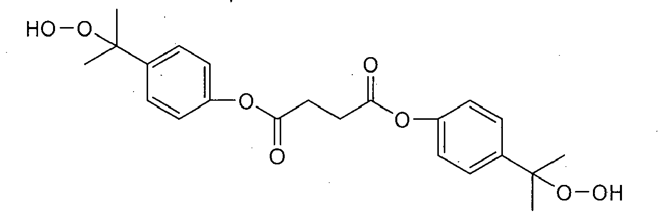

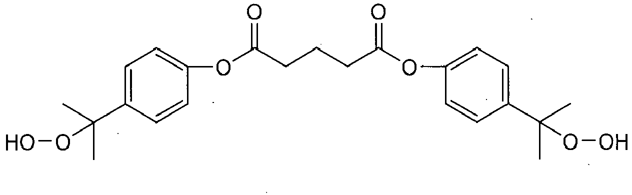

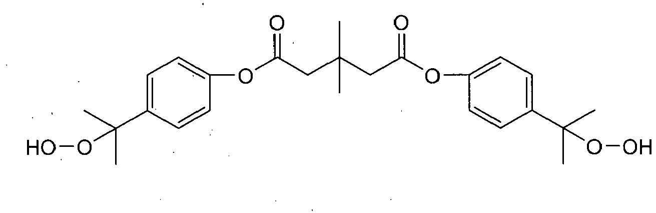

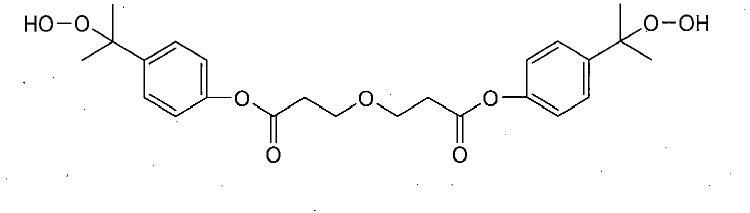

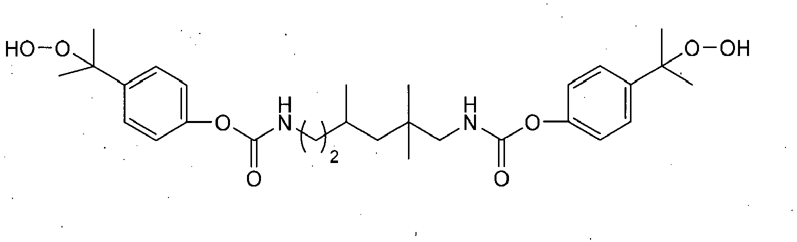

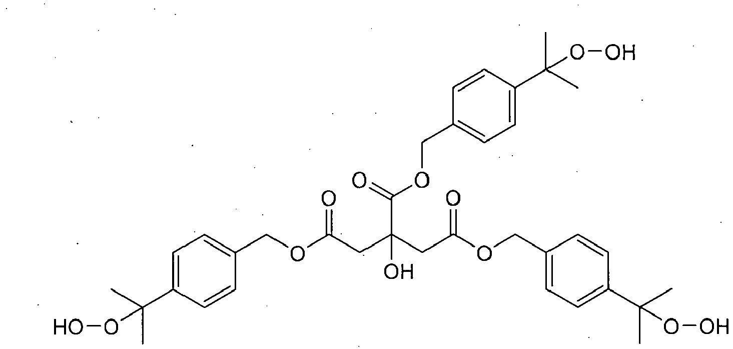

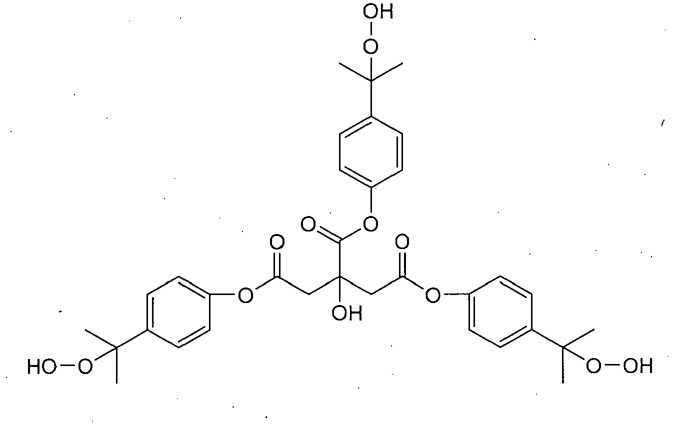

The hydroperoxide derivatives of Formula II have a low odor and high storage stability at room temperature. They are particularly suitable for the production of low-odor dental materials. The dental materials of the present invention can contain one or more hydroperoxides.

The materials according to the invention contain at least one **polyfunctional radically polymerizable monomer without acid group.** It was found that polyfunctional monomers yield a high polymerization rate and mechanical properties suitable for dental applications.

Polyfunctional monomers are understood to be compounds with two or more, preferably 2 to 4 and in particular 2 radically polymerizable groups. Monofunctional monomers accordingly have only one radically polymerizable group. Polyfunctional monomers have crosslinking properties and are therefore also referred to as crosslinking monomers. Preferred radically polymerizable groups are (meth)acrylate, (meth)acrylamide and vinyl groups. Most preferred polyfunctional monomers are polyfunctional (meth)acrylates and in particular methacrylates.

Preferred polyfunctional (meth)acrylates are di- and polyfunctional (meth)acrylates, more preferably bisphenol-A-dimethacrylate, bis-GMA (an addition product of methacrylic acid and bisphenol-A-diglycidyl ether), ethoxy- or propoxylated bisphenol-A-dimethacrylates, such as the bisphenol A dimethacrylate SR-348c (Sartomer) with 3 ethoxy groups or 2,2-bis[4-(2-methacryloyloxypropoxy)phenyl]propane, urethanes of 2-(hydroxymethyl)-acrylic acid and diisocyanates, such as 2,2,-4-trimethylhexamethylene diisocyanate or isophoronic diisocyanate, UDMA (an addition product of 2-hydroxyethyl methacrylate and 2,2,4-trimethylhexamethylene-1,6-diisocyanate), 2-{[(2-(methacryloyloxy)ethoxy)carbonyl]amino} ethyl methacrylate (V-837), tetramethylxylylene diurethane ethylene glycol di(meth)acrylate and tetramethylxylylene diurethane ethylene glycol di(meth)acrylate, respectively. Tetramethylxylylene diurethane-2-methylethylene glycoldi(meth)acrylate (V380), di-, tri- or tetraethylene glycol dimethacrylate, trimethylol propanetrimethacrylate, pentaerythritol tetramethacrylate and glycerol di- and trimethacrylate, 1,4-butanediol dimethacrylate, 1,10-decanediol dimethacrylate (D3MA), bis(methacryloyloxymethyl)tricyclo-[5.2.1.02,6]decane (DCP), polyethylene glycol or polypropylene glycol dimethacrylates, such as polyethylene glycol 200-dimethacrylate or polyethylene glycol 400-dimeth¬acrylate (PEG-200- or PEG-400-DMA) or 1,12-dodecanediol dimethacrylate. UDMA, V-380, triethylene glycol dimethacrylate (TEGDMA) and PEG-400-DMA (NK ester 9G) are particularly preferred.

The monomer V380 has the following formula:

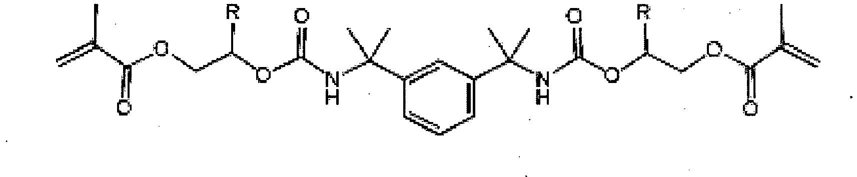

In this formula, the R groups are each independently H or CH₃, and the R residues may have the same meaning or different meanings. Preferably, a mixture is used which contains molecules in which both residues are H, molecules in which both residues are CH₃, and molecules in which one residue is H and the other residue is CH₃, the ratio of H to CH₃ preferably being 7:3. Such a mixture is obtainable, for example, by reaction of 1,3-bis(1-iso-cyanato-1-methylethyl)benzene with 2-hydroxypropyl methacrylate and 2-hydroxyethyl methacrylate.

The catalyst paste of the materials according to the invention contains at least one radically polymerizable **monomer and/or oligomer with one or more acid groups.** It was found that the presence of monomers and/or oligomers with acid groups significantly improves the reactivity of the materials and leads to better mechanical properties (flexural strength and flexural modulus). By selecting the content of acidic monomers, the polymerization time (e.g. processing time for luting materials) can be precisely adjusted. In addition, acidic monomers and oligomer can impart adhesive properties to the materials, especially with dentine and enamel. Without wishing to be bound by any theory, it is assumed that the acidic monomer or oligomer protonates the dihydropyridine derivative of Formula (I) and that the resulting salt then reacts with the oxidizing agent, leading to the formation of radicals that can initiate polymerization.

The compositions according to the invention preferably contain monomers with and monomers without acid group in a weight ratio of from 1:3 to 1:99, more preferably from 1:5 to 1:65 and most preferably from 1:7 to 1:40.

Radically polymerizable monomers or oligomers with an acid group are understood to mean monomers or oligomers which, in addition to at least one radically polymerizable group, contain at least one acid group, preferably a carboxylic acid group, phosphoric acid ester group and/or phosphonic acid group, with phosphonic acid groups and in particular phosphoric acid ester groups, especially monohydrogen phosphate or dihydrogen phosphate groups, being particularly preferred. Preferred radically polymerizable groups are (meth)acrylate groups and in particular methacrylate groups. Thus, monomers comprising at least one (meth)acrylate group and at least one carboxylic acid group, preferably at least one phosphonic acid group or phosphoric acid ester group are preferred. Polymerizable dihydrogen phosphates are most preferred.

Such monomers or oligomers are also referred to herein as acidic monomers or oligomers, respectively. Accordingly, radically polymerizable monomers without an acid group are monomers that do not contain an acid group and, in particular, do not contain a carboxylic acid group, phosphonic acid group, phosphoric acid group, or phosphoric acid ester group.

Preferred acidic monomers containing a COOH group are 4-(meth)acryloyl-oxyethyltrimellitic acid, 10-methacryloyloxydecylmalonic acid, N-(2-hydroxy-3-methacryloyloxypropyl)-N-phenylglycine, 4-vinylbenzoic acid and 2-carboxyethyl acrylate (CEA).

It was found that the increase in reactivity of the compositions depends on the pKa value of the acidic compound. The lower the pKa of the acidic compound, the greater the increase in reactivity. Acidic monomers having a pKa of 5 or less, preferably 4 or less and most preferably 3 or less are preferred. Particularly preferred are acidic monomers having a pKa of 1 to 5, more preferably 1 to 4 and most preferably 1 to 3. For this reason, monomers comprising at least one phosphoric acid ester group and/or phosphonic acid group are preferred.

Preferred radically polymerizable phosphonic acids are 4-vinylbenzylphosphonic acid, 2-[4-(dihydroxyphosphoryl)-2-oxa-butyl]-acrylic acid and its esters, such as its ethyl ester, 2-[4-(di-hydroxyphosphoryl)-2-oxa-butyl]acrylate (EDOBA) and 2,4,6-trimethylphenyl 2-[4-(dihydroxyphosphoryl)-2-oxa-butyl]acrylate. EDOBA is most preferred.

Preferred radically polymerizable monohydrogen phosphates are bis[(2-methacryloyloxy)-ethyl] phosphate and bis[(6-methacryloyloxy)hexyl] phosphate.

Preferred radically polymerizable dihydrogen phosphates are 2-methacryloyloxyethylphenyl hydrogen phosphate, 10-methacryloyloxydecyl dihydrogen phosphate (MDP), glycerine dimethacrylate dihydrogen phosphate, dipentaerythritol pentamethacryloyloxy phosphate. MDP is most preferred.

Acidic, **radically polymerizable oligomers** comprise at least one acid group, preferably a carboxyl group, and at least one radically polymerizable group, preferably a (meth)acrylate group and in particular methacrylate group. Oligomers are defined as polymers with a degree of polymerization Pn of 2 to 100 (Pn = Mn / Mu; Mn: number average polymer molecular weight, Mu: molecular weight of the monomer unit). Acidic oligomers can be added instead of, or preferably in addition to, one or more acidic monomers.

Preferred polymerizable acidic oligomers are oligomeric carboxylic acids, such as polyacrylic acid, preferably with a number average molecular weight Mn of less than 7200 g/mol, preferably less than 7000 g/mol and particularly preferably less than 6800 g/mol, where Mn is preferably in a range from 800 to 7200 g/mol, particularly preferably from 500 to 7000 g/mol and most preferably from 500 to 6800 g/mol. Oligomeric carboxylic acids with (meth)acrylate groups are particularly preferred. These are obtainable, for example, by reacting oligomeric polyacrylic acid with glycidyl methacrylate or 2-isocyanatoethyl methacrylate.

The number average molecular weight Mn is preferabyl determined by gel permeation chromatography (GPC). Gel permeation chromatography (GPC) is a relative method in which the molecules are separated on the basis of their size, or more precisely on the basis of their hydrodynamic volume. The absolute molar mass is determined by calibration with known standards. Preferably, narrowly distributed polystyrene standards are used as calibration standards. These are commercially available. Styrene-divinylbenzene columns are used as separation media and tetrahydrofuran (THF) as eluent. Styrene-divinylbenzene columns are suitable for organic soluble synthetic polymers. The measurement is carried out with dilute solutions (0.05 - 0.2 wt.%) of the polymers to be investigated.

Alternatively, the number-average molar mass can be determined by the known methods of freezing point depression (cryoscopy), boiling point elevation (ebullioscopy) or from vapor pressure depression (vapor pressure osmometry). These are absolute methods that do not require calibration standards. Concentration series of 4 to 6 diluted polymer solutions with concentrations of 0.005 to 0.10 mol/kg are examined and then the measured values are extrapolated to a concentration of 0 mol/kg.

Preferably, the materials of the present invention additionally contain a **monofunctional radically polymerizable monomer** without an acid group. Preferred monofunctional (meth)acrylates are benzyl, tetrahydrofurfuryl or isobornyl (meth)acrylate, p-cumylphenoxyethylene glycol methacrylate (CMP-1E) and 2-([1,1'-biphenyl]-2-oxy)ethyl methacrylate (PEM), tricyclodecane methyl (meth)acrylate, 2-(2-biphenyloxy)ethyl(meth)-acrylate. CMP-1E and PEM are particularly preferred.

The compositions of the present invention comprise at least 50 % by weight, preferably at least 60 % by weight and most preferably at least 70 % by weight, of polyfunctional radically polymerizable monomers or monomers and oligomers, based on the total amount of polymerizable monomers and oligomers. The total amount of radically polymerizable monomers and oligomers comprises all radically polymerizable monomers and oligomers contained in the base paste and in the catalyst paste. Both the group of polyfunctional radically polymerizable monomers or monomers and oligomers and the total amount of radically polymerizable monomers and oligomers include compounds with and without an acid group.

The dihydropyridine derivatives according to Formula (I), in combination with an oxidizing agent, form a redox initiator system that initiates the radical polymerization of (meth)acrylates. It was found that compositions containing a dihydropyridine derivative according to Formula (I) and an oxidizing agent, preferably a (hydro)peroxide, in combination with an acidic monomer and/or oligomer, have excellent properties (mechanical properties and working time), do not have a bitter taste and are biocompatible. Such compositions are therefore particularly suitable as dental materials, preferably dental cements, temporary filling materials and two component filling composites.

It has also been found that dihydropyridine derivatives according to Formula (I) in combination with an oxidizing agent, preferably a hydroperoxide, can initiate the radical polymerization of (meth)acrylates even in the absence of thiourea derivatives and other reducing agents such as amines, (thio)barbituric acid derivatives, ascorbic acid, ascorbic acid derivatives, and/or sulfinates. It is therefore possible to produce dental materials which contain no thiourea or thiourea derivatives and which preferably also contain no other reducing agents, in particular no amines, (thio)barbituric acid derivatives, ascorbic acid, ascorbic acid derivatives and sulfinates.

According to a further preferred embodiment, the materials according to the invention contain at least one **transition metal compound** in addition to the dihydropyridine of Formula (I) and the oxidizing agent. The transition metal compound is added to the base paste. It has been found that the addition of a transition metal compound yields materials which have significantly improved mechanical properties after hardening.

Transition metal compounds preferred according to the invention are compounds which are derived from transition metals which have at least two stable oxidation states. Compounds of the elements copper, cobalt, nickel, vanadium and manganese are particularly preferred. These metals have the following stable oxidation states: Cu(I)/Cu(II), Co(II)/Co(III), Ni(II)/Ni(III), V(III)/V(IV), V(IV)/V(V), Mn(II)/Mn(III). Materials which contain at least one copper compound are particularly preferred. In all cases, those compounds in which the respective transition metal is present in its most stable oxidation state are preferred. Compounds of Cu²⁺ are thus most preferred. The compositions of the present invention preferably do not contain iron compounds. It was found that compositions comprising iron compound are less stable in air.

The transition metals are preferably used in the form of their salts. Preferred salts are the nitrates, acetates, 2-ethylhexanoates and halides, wherein chlorides are particularly preferred.

The transition metals can furthermore advantageously be used in complexed form, wherein complexes with chelate-forming ligands are particularly preferred. Preferred simple ligands for complexing the transition metals are 2-ethylhexanoate and THF. Preferred chelate-forming ligands are 2-(2-aminoethylamino)ethanol, aliphatic amines, particularly preferably 1,1,4,7,10,10-hexamethyltriethylenetetramine (HMTETA), N,N,N',N",N"-pentamethyldiethylenetriamine (PMDETA), tris[2-(dimethylamino)ethyl]amine (Me₆TREN), N,N,N',N'-tetramethylethylenediamine (TMEDA), 1,4,8,11-tetraaza-1,4,8,11-tetramethyl-cyclotetradecane (Me4CYCLAM), diethylenetriamine (DETA), triethylenetetramine (TETA) and 1,4,8,11-tetra-azacyclotetradecane (CYCLAM); pyridine-containing ligands, particularly preferably N,N,N',N'-tetrakis(2-pyridylmethyl)ethylenediamine (TPEN), N, N-bis(2-pyridylmethyl)amine (BPMA), N,N-bis(2-pyridylmethyl)octylamine (BPMOA), 2,2'-bipyridine and 8-hydroxyquinoline. Most particularly preferred ligands are acetylacetone, dimethylglyoxime and 1,10-phenanthroline. Metal complexes that are soluble in the respective monomer mixture are particularly preferred.

In the case of electrically neutral ligands, the charge of the transition metal ions must be balanced by suitable counterions. For this, the above-named ions which are used to form salts are preferred wherein acetates and chlorides are particularly preferred. Chlorides and complexes are characterized by a relatively good solubility in monomers, which are used to prepare dental materials.

Instead of the transition metal complexes, non-complex salts of the transition metals in combination with complex-forming organic compounds can be used to prepare the dental materials, preferably in combination with the above-named chelate-forming compounds. The organic ligands form the catalytically active complexes when mixed with the transition metal salts. The use of such combinations of transition metal salts and organic ligands is preferred.

Preferred copper salts are CuCI, CuBr, CuCl₂, CuBr₂, CuI₂, Cu(II) carboxylates (e.g. of acetic acid or 2-ethylhexanoic acid). Preferred copper complexes are complexes with the ligands acetylacetone, phenanthroline (e.g. 1,10-phenanthroline (phen)), the aliphatic amines, such as e.g. 1,1,4,7,10,10-hexamethyltriethylenetetramine (HMTETA), N,N,N',N",N"-pentamethyl-diethylenetriamine (PMDETA), tris[2-(dimethylamino)ethyl]amine (Me₆TREN).

In addition to the redox initiator system, the compositions according to the invention can advantageously contain an **initiator for the radical photopolymerization.** Such compositions are dual-curing, i.e. they can be cured both chemically and by light. Photoinitiators can be added to the catalyst paste, the base paste or both.

Preferred photoinitiators are alpha-diketones derivatives, preferably camphorquinone (CQ), 9,10-phenanthrenequinone, 1-phenylpropane-1,2-dione, 2,2-dimethoxy-2-phenylacetophenone, diacetyl or 4,4'-dichlorobenzil or derivatives thereof. Camphorquinone (CQ), 2,2-dimethoxy-2-phenylacetophenone and mixtures thereof are most particularly preferred. Alpha-diketones are preferably used in combination with tertiary amines as coinitiator. Preferred coinitiators are 4-(dimethylamino)benzoic acid ester (EDMAB), N,N-dimethylaminoethyl methacrylate, N,N-dimethyl-sym.-xylidine and triethanolamine. Bimolecular photoinitiators, i.e. photoinitiators that require a second component to reach their optimal activity, are referred to as Norrish type 2 photoinitiators.

According to the present invention, compositions that do not contain amines are preferred. For this reason, Norrish type 1 photoinitiators are particularly preferred as type I Norrish photoinitiators do not require an amine component. The materials according to the invention preferably contain one or more Norrish type 1 photoinitiator, more preferably such a photoinitiator which is active in a wavelength range of from 400 to 500 nm.

Preferred Norrish type 1 photoinitiators are monoacyl- or bisacylphosphine oxides, diacyldialkylgermanium and tetraacylgermanium compounds as well as tetraacylstannanes. Particularly preferred monomolecular photoinitiators are 2,4,6-trimethylbenzoyl diphenylphosphine oxide, bis(2,4,6-trimethylbenzoyl) phenylphosphine oxide, Ethyl (2,4,6-trimethylbenzoyl) phenylphosphinate (TPO-L), dibenzoyldiethylgermane, bis(4-methoxybenzoyl)diethylgermanium (MBDEGe, trade name Ivocerin), tetrabenzoylgermane, tetrakis(o-methylbenzoyl)germane, tetrakis(mesitoyl)stannane and mixtures thereof.

Moreover, mixtures of the different photoinitiators can also be used, such as e.g. bis(4-methoxybenzoyl)diethylgermane or tetrakis(o-methylbenzoyl)germane in combination with camphorquinone and 4-dimethylaminobenzoic acid ethyl ester.

The dental materials according to the invention can moreover advantageously contain one or more **organic or inorganic fillers.** Particulate fillers are preferred. Filler-containing compositions are particularly suitable as dental fixing cements or filling composites. Fillers can be added to the catalyst paste, the base paste or both.

Preferred inorganic fillers are oxides, such as SiO₂, ZrO₂ and TiO₂ or mixed oxides of SiO₂, ZrO₂, ZnO and/or TiO₂, nanoparticulate or microfine fillers, such as pyrogenic silica or precipitated silica, glass powders, such as quartz, glass ceramic, borosilicate or radiopaque glass powders, preferably barium or strontium aluminium silicate glasses, and radiopaque fillers, such as ytterbium trifluoride, tantalum(V) oxide, barium sulfate or mixed oxides of SiO₂ with ytterbium(III) oxide or tantalum(V) oxide. The dental materials according to the invention can furthermore contain fibrous fillers, nanofibres, whiskers or mixtures thereof.

Preferably, the oxides have a particle size of from 0.010 to 15 µm, the nanoparticulate or microfine fillers preferably have a particle size of from 10 to 300 nm, the glass powders preferably have a particle size of from 0.01 to 15 µm, more preferably of from 0.2 to 1.5 µm, and the radiopaque fillers preferably have a particle size of from 0.2 to 5 µm.

Particularly preferred fillers are mixed oxides of SiO₂ and ZrO₂, with a particle size of from 10 to 300 nm, glass powders with a particle size of from 0.2 to 1.5 µm, in particular radiopaque glass powders, preferably of barium or strontium aluminium silicate glasses, and radiopaque fillers with a particle size of from 0.2 to 5 µm, in particular ytterbium trifluoride and/or mixed oxides of SiO₂ with ytterbium(III) oxide.

Polymer particles, in particular particles of crosslinked organic polymers, are preferred as organic fillers. Polymer particles can be obtained by curing one or more monomers and then grinding the cured mixture, or by suspension polymerization. The mixtures can be cured by light irradiation or thermally. Radically polymerizable monomers and in particular the monomers listed above are preferred as monomers. Dimethacrylates, specifically bis-GMA, UDMA, TMX-UDMA, DCP, D3MA and mixtures thereof, are most particularly preferred.

Polymer particles prepared by grinding exhibit a splintery appearance under the microscope and are therefore also referred to as splinter polymerizates or splinter polymers. Pearl polymerizates obtained by suspension polymerization in contrast are characterized by spherical particles. Splinter polymerizates are preferred as organic fillers.

In addition, so-called composite fillers, i.e. organic-inorganic fillers, are preferred as fillers. These are also referred to as isofillers. These are fragmentary polymers which in turn contain an inorganic filler, preferably pyrogenic SiO₂ and/or ytterbium trifluoride. Preferred are polymers based on dimethacrylates. For the production of isofillers, the inorganic filler(s) is/are incorporated, for example, into a dimethacrylate resin matrix, and the resulting composite paste is subsequently thermally polymerized and then ground.

A composite filler preferred according to the invention can be prepared, for example, by thermally curing a mixture of Bis-GMA (8.80% by weight), UDMA (6.60% by weight), 1,10-decanediol dimethacrylate (5.93% by weight), dibenzoyl peroxide + 2,6-di-tert butyl-4-methylphenol (together 0.67 wt.%), glass filler (average grain size 0.4 µm; 53.0 wt.%) and YbF₃ (25.0 wt.%) and subsequent grinding of the cured material to the desired grain size. Another composite filler preferred according to the invention is a hot-cured composite filler ground to the desired grain size and prepared from UDMA (23.58 % by weight), 1,10-decanediol dimethacrylate (5.92 % by weight), dibenzoyl peroxide (0.50 % by weight), fumed silica Aerosil OX50 (50.0 % by weight) and YbF₃ (20.0 % by weight). All percentages refer to the total mass of the composite filler.

So-called inert fillers can also be used as fillers. These are glass fillers whose surface is coated with a diffusion barrier layer, e.g. on a sol-gel basis, or with a polymer layer, e.g. of PVC. Preferred fillers are those described in EP 2 103 296 A1.

Unless otherwise stated, all particle sizes are weight-average particle sizes, wherein the particle-size determination in the range of from 0.1 µm to 1000 µm is effected by means of static light scattering, preferably using an LA-960 static laser scattering particle size analyzer (Horiba, Japan). Here, a laser diode with a wavelength of 655 nm and an LED with a wavelength of 405 nm are used as light sources. The use of two light sources with different wavelengths makes it possible to measure the entire particle-size distribution of a sample in only one measurement pass, wherein the measurement is carried out as a wet measurement. For this, a 0.1 to 0.5% aqueous dispersion of the filler is prepared, and the scattered light thereof is measured in a flow cell. The scattered-light analysis for calculating particle size and particle-size distribution is effected in accordance with the Mie theory according to DIN/ISO 13320: 2020.

Particle sizes smaller than 0.1 µm are preferably determined by means of dynamic light scattering (DLS). The measurement of the particle size in the range of from 5 nm to 0.1 µm is preferably effected by dynamic light scattering (DLS) of aqueous particle dispersions, preferably with a Malvern Zetasizer Nano ZS (Malvern Instruments, Malvern UK) with a He-Ne laser with a wavelength of 633 nm, at a scattering angle of 90° at 25° C.

Particle sizes smaller than 0.1 µm can also be determined by means of SEM or TEM spectroscopy. The transmission electron microscopy (TEM) is preferably carried out with a Philips CM30 TEM at an accelerating voltage of 300 kV. For the preparation of the samples, drops of the particle dispersion are applied to a 50 Å thick copper grid (mesh size 300), which is coated with carbon, and then the solvent is evaporated.

The light scattering decreases as the particle size decreases, but fillers with a small particle size have a greater thickening action. The fillers are divided according to their particle size into macrofillers and microfillers, wherein fillers with an average particle size of from 0.2 to 10 µm are called macrofillers and fillers with an average particle size of from approx. 5 to 100 nm are called microfillers. Macrofillers are obtained e.g. by grinding e.g. quartz, radiopaque glasses, borosilicates glasses or glass ceramics and usually consist of splintery parts. Microfillers such as mixed oxides can be prepared e.g. by hydrolytic co-condensation of metal alkoxides.

To improve the bond between the filler particles and the crosslinked polymerization matrix, the fillers are preferably surface-modified, particularly preferably by silanization, most particularly preferably by radically polymerizable silanes, in particular with 3-methacryloyloxypropyltrimethoxysilane. For the surface-modification of non-silicate fillers, e.g. of ZrO₂ or TiO₂, functionalized acidic phosphates, such as e.g. 10-methacryloyloxydecyl dihydrogen phosphate can also be used.

The compositions according to the invention preferably contain from 20% to 90% by weight, more preferably from 40% to 80% by weight and most preferably from 50% to 80% by weight of fillers, in each case based on the total mass of the composition.

The compositions according to the invention may also contain further **additives,** in particular stabilizers, such as e.g. polymerization stabilizers, dyes, microbiocidal active ingredients, fluoride-ion-releasing additives such as fluoride salts in particular NaF, ammonium fluoride, or fluorosilanes, optical brighteners, fluorescent agents, plasticizers, and/or UV absorbers

The materials according to the invention comprise two physically separated components which are mixed with each other for use. The first component (catalyst paste) contains the oxidizing agent, and the second component (base paste) contains the dihydropyridine derivative of Formula (I) and optionally the transition metal compound.

For application, the catalyst and base pastes are preferably mixed in approximately equal proportions, preferably in a volume ratio of 1:1. They are therefore particularly suitable for use with a double-push syringe. The hardening reaction is initiated by mixing base and catalyst pastes.

Double-shear syringes have two separate cylindrical chambers for holding the base paste and catalyst paste. The components are pressed out of the chambers simultaneously by two pistons connected to each other and are preferably forced through a mixing cannula and mixed together therein. For pressing out the pastes, the syringe can be inserted into a so-called hand dispenser, which facilitates handling of the syringes.

The catalyst paste preferably comprises:
(a) 4 to 78 % by weight of at least one polyfunctional radically polymerizable monomer without an acid group,
(b) 0 to 40 % by weight of at least one monofunctional radically polymerizable monomer without an acid group,
(c) 0.1 to 40 % by weight of at least one radically polymerizable monomer and/or oligomer, preferably a monomer, with an acid group,
(d) 0.05 to 8 % by weight of at least one peroxide and/or hydroperoxide,
   0 to 2 % by weight of one or more photoinitiators,
(j) 20 to 90 % by weight of at least one filler,
(k) 0 to 2 % by weight of one or more additives,
based on the total mass of the catalyst paste.

The base paste preferably comprises:
(e) 9 to 79 % by weight of at least one polyfunctional radically polymerizable monomer without an acid group,
(f) 0 to 40 % by weight of at least one monofunctional radically polymerizable monomer without an acid group,
(g) 0.05 to 5 % by weight of at least one dihydropyridine derivative according to Formula (I),
(h) 1 to 500 ppm of at least one transition metal compound,
(i) 0 to 2 % by weight of one or more photoinitiators,
(j) 20 to 90 % by weight of at least one filler,
(k) 0 to 2 % by weight of one or more additives
based on the total mass of the base paste.

According to a more preferred embodiment of the invention the catalyst paste comprises:
(a) 10 to 58 % by weight of at least one polyfunctional radically polymerizable monomer without an acid group,
(b) 0 to 30 % by weight of at least one monofunctional radically polymerizable monomer without an acid group,
(c) 0.3 to 15 % by weight of at least radically polymerizable monomer and/or oligomer, preferably a monomer, with an acid group,
(d) 0.25 to 5 % by weight of at least one peroxide and/or hydroperoxide,
(i) 0 to 1.5 % by weight of one or more photoinitiators,
(j) 40 to 80 % by weight of at least one filler,
(k) 0 to 1 % by weight of one or more additives,
based on the total mass of the catalyst paste,
and the base paste comprises:
(e) 19 to 60 % by weight of at least one polyfunctional radically polymerizable monomer without an acid group,
(f) 0 to 30 % by weight of at least one monofunctional radically polymerizable monomer without an acid group
(g) 0.3 to 3 % by weight of at least one dihydropyridine derivative according to Formula (I),
(h) 10 to 200 ppm of at least one transition metal compound,
(i) 0 to 1.5 % by weight of one or more photoinitiators,
(j) 40 to 80 % by weight of at least one filler,
(k) 0 to 1 % by weight of one or more additives,
based on the total mass of the base paste.

According to a particularly preferred embodiment of the invention the catalyst paste comprises:
(a) 15 to 49 % by weight of at least one polyfunctional radically polymerizable monomer without an acid group,
(b) 0 to 25 % by weight of at least one monofunctional radically polymerizable monomer without an acid group,
(c) 0.5 to 10 % by weight of at least one radically polymerizable monomer and/or oligomer, preferably a monomer, with an acid group,
(d) 0.5 to 3 % by weight of at least one peroxide and/or hydroperoxide,
(i) 0 to 1.0 % by weight of one or more photoinitiators,
(j) 50 to 80 % by weight of at least one filler,
(k) 0 to 1 % by weight of one or more additives,
based on the total mass of the catalyst paste,
and the base paste comprises:
(e) 19 to 49 % by weight of at least one polyfunctional radically polymerizable monomer without an acid group,
(f) 0 to 25 % by weight of at least one monofunctional radically polymerizable monomer without an acid group
(g) 0.3 to 2 % by weight of at least one dihydropyridine derivative according to Formula (I),
(h) 10 to 100 ppm of at least one transition metal compound,
(i) 0 to 1.0 % by weight of one or more photoinitiators,
(j) 50 to 80 % by weight of at least one filler,
(k) 0 to 1 % by weight of one or more additives,
based on the total mass of the base paste.

In all cases, the compositions defined above comprise at least 50 % by weight, preferably at least 60 % by weight and most preferably at least 70 % by weight, of polyfunctional radically polymerizable monomers and/or oligomers, based on the total amount of polymerizable monomers and/or oligomers.

The pastes are preferably blended such that catalyst and base pastes can be used in a volume ratio of 1:1.

The dental materials according to the invention are characterized in that they do not have a bitter taste after hardening and have excellent biocompatibility. Moreover, they have good curing characteristics, i.e. they have an advantageous processing time in combination with an advantageous curing time. Furthermore, after hardening, the materials have mechanical properties which are comparable with materials based on established redox systems, such as e.g. a mixture of acetyl thiourea and cumene hydroperoxide (CHP), without restrictions.

The compositions according to the invention are particularly suitable as dental materials, in particular as dental cements, two component filling composites and temporary filling materials. They are particularly suitable as dental materials for intraoral use by the dentist for the restoration of damaged teeth, i.e. for therapeutic use, especially as dental cements, layering or veneering materials, restorative composites, and especially as luting cements. However, they can also be used non-therapeutically (extraorally), for example in the production or repair of dental restorations, such as prostheses, artificial teeth, inlays, onlays, crowns and bridges.

For the treatment of damaged teeth, these are preferably prepared in a first step by the dentist. Subsequently, at least one composition according to the invention is applied to or into the prepared tooth. After this, the composition can be cured directly, preferably by irradiation with light of a suitable wavelength, for example in the restoration of cavities. Alternatively, a dental restoration, for example an inlay, on-lay, veneer, crown, bridge, framework or dental ceramic, is placed in or applied to the prepared tooth. Subsequent curing of the composition is preferably by light and/or by self-curing. The dental restoration is attached to the tooth in this process.

In the repair of dental restorations, the compositions according to the invention are baked onto the restoration to be repaired, for example to repair gaps or to bond fragments, and then cured.

The invention is explained in more detail below with reference to examples.

### Examples

### Example 1

### Synthesis of 3,5-diethyl-1,2-dihydro-1-phenyl-2-propylpyridine DHP1

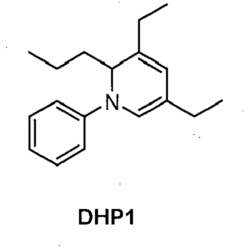

Aniline (37.25 g; 0.40 mol) was slowly added to a cooled dispersion of n-butyraldehyde (135.27 g; 1.88 mol) in water (54.00 g; 3.00 mol) and acetic acid (6.00 g; 0.10 mol) at 20 °C. The reaction mixture was heated to 42 °C for 1 h and to 75 °C for 5 h. The phases were separated, and the organic phase was subjected to vacuum distillation. The major fraction containing 70% of DHP1 (b.p.: 63 °C/0.09 mbar) was further purified by column chromatography (SiO₂, n-hexane), affording 45.98 g (0.18 mol; 45%) of pure DHP1 (CAS Registry Number 34562-31-7) as a yellow liquid.

¹H NMR (CDCl₃, 400 MHz): δ = 0.90 (t, 3H; J = 7.3 Hz; CH₃), 1.05 (t, 3H; *J* = 7.3 Hz; CH₃), 1.10 (t, 3H; *J* = 7.3 Hz; CH₃), 1.37 - 1.46 (m, 2H; CH₂); 1.54 - 1.61 (m, 2H; CH₂); 1.97 - 2.26 (m, 4H; CH₂); 4.27 (t, 1H; *J* = 6.6 Hz; CH), 5.68 (s, 1H; =CH), 6.07 (s, 1H; =CH), 6.83 (t, 1H; *J* = 7.3 Hz; ArH), 6.92 (d, 2H; J = 8.1 Hz; ArH), 7.21 - 7.28 (m, 2H; ArH).

¹³C NMR (CDCl₃, 100.6 MHz): δ = 12.0 (CH₃), 14.4 (CH₃), 14.5 (CH₃), 19.4 (CH₂), 25.6 (CH₂), 27.7 (CH₂), 33.8 (CH₂), 58.6 (CH), 114.8 (=CH), 118.1 (=CH), 119.1 (=CH), 120.1 (=C), 120.2 (=CH), 129.0 (=CH), 134.2 (=C), 146.3 (=C).

### Example 2

### Synthesis of 3,5-diethyl-1,2-dihydro-1-(4-methoxyphenyl)-2-propylpyridine DHP2

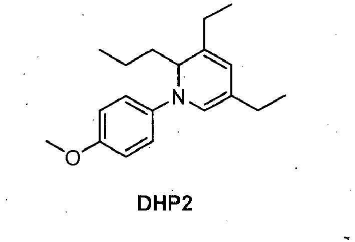

p-Anisidine (12.53 g; 0.10 mmol) was added in portions to a cooled dispersion of *n-*butyraldehyde (54.23 g; 0.75 mol) in water (21.60 g; 1.20 mol) and acetic acid (2.40 g; 0.04 mol) at 10 °C. The reaction mixture was heated to 42 °C for 1 h and to 75 °C for 5 h. The phases were separated, and the organic phase was subjected to vacuum distillation. The major fraction containing 72% of DHP2 (b.p.: 130 °C/0.08 mbar) was further purified by column chromatography (SiO₂, n-hexane), affording 12.56 g (0.18 mol; 44%) of pure DHP2 (CAS Registry Number 145191-18-0) as a yellow liquid.

¹H NMR (CDCl₃, 400 MHz): δ = 0.91 (t, 3H; J = 7.2 Hz; CH₃), 1.04 (t, 3H; J = 7.2 Hz; CH₃), 1.09 (t, 3H; J = 7.2 Hz; CH₃), 1.36 - 1.47 (m, 2H; CH₂), 1.51 - 1.61 (m, 2H; CH₂), 1.96 - 2.25 (m, 4H; CH₂), 3.76 (s, 3H; OCH₃), 4.18 (t, 1H; *J* = 6.3 Hz; CH), 5.68 (s, 1H; =CH), 6.00 (s, 1H; =CH), 6.79 - 6-90 (m, 4H; ArH).

¹³C NMR (CDCl₃, 100.6 MHz): δ = 12.0 (CH₃), 14.5 (CH₃), 14.6 (CH₃), 19.4 (CH₂), 25.6 (CH₂), 27.7 (CH₂), 34.0 (CH₂), 55.6 (OCH₃), 59.6 (CH), 114.5 (=CH), 116.6 (=CH), 118.0 (=CH), 118.9 (=C), 120.9 (=CH), 132.8 (=C), 140.8 (=C), 153.3 (=C).

### Example 3

### Step 1: Synthesis of 1-(4-chlorophenyl)-3,5-diethyl-2-propyl-1,2-dihydropyridine

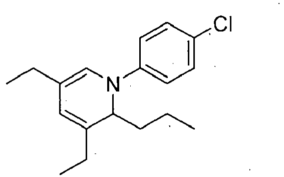

n-Butyraldehyde (33.89 g; 0.47 mol) was slowly added to a mixture of acetic acid (1.50 g, 25.0 mmol), 4-chloroaniline (12.76 g, 0.20 mol), and water (13.50 g). The reaction mixture was heated to 45 °C for 1 h and to 75 °C for 44 h. Upon cooling to RT, n-heptane (100 mL) and water (100 mL) were added, and the phases were separated. The organic phase was washed with water (2x 100 mL) and brine (100 mL), dried over anhydrous sodium sulfate, filtered, and the solvent was evaporated. The crude product was purified by column chromatography (SiO₂, n-heptane/ethyl acetate 9:1), affording 13.80 g (47.6 mmol; 48%) of the desired product as a yellow liquid.

¹H NMR (CDCl₃, 400 MHz): δ = 0.90 (t, 3H; *J* = 7.3 Hz; CH₃), 1.05 (t, 3H; J = 7.4 Hz; CH₃), 1.09 (t, 3H; *J* = 7.4 Hz; CH₃), 1.33 - 1.44 (m, 2H; CH₂), 1.51 - 1.61 (m, 2H; CH₂), 1.96 - 2.26 (m, 4H; CH₂), 4.19 (t, 1H; *J* = 6.6 Hz; CH), 5.68 (s, 1H; =CH), 5.99 (s, 1H; =CH), 6.80 - 6.85 (m, 2H; ArH), 7.15 - 7.21 (m, 2H; ArH).

¹³C NMR (CDCl₃, 100.6 MHz): δ = 11.9 (CH₃), 14.3 (CH₃), 14.5 (CH₃), 19.4 (CH₂), 25.5 (CH₂), 27.6 (CH₂), 33.7 (CH₂), 58.8 (CH), 115.8 (=CH), 118.1 (=CH), 119.7 (=CH), 120.8 (=C), 123.9 (=C), 128.9 (=CH), 134.6 (=C), 145.0 (=C).

### Step 2: Synthesis of 3,5-diethyl-1-(4-nitrophenyl)-2-propyl-1,2-dihydropyridine DHP3

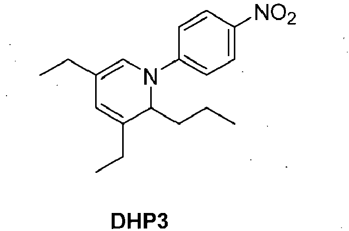

A flask was charged with 1-(4-chlorophenyl)-3,5-diethyl-2-propyl-1,2-dihydropyridine (4.40 g, 15.2 mmol), tris(dibenzylideneacetone)dipalladium(0) (0.07 g, 0.08 mmol), 2-(di-*tert*.-butylphosphino)-2",4",6"-triisopropyl-3,6-dimethoxy-1,1"-biphenyl (0.09 g, 0.18 mmol). And sodium nitrite (2.09 g, 30.4 mmol). The flask was evacuated and placed under argon. Tris(2-(2-methoxyethoxy)ethyl)amine (0.25 g, 0.76 mmol) and 2-methyl-2-butanol (30 mL) were added *via* syringe and the reaction mixture was heated to 130 °C for 24 h. Upon cooling to room temperature, ethyl acetate (100 mL) and water (50 mL) were added, and the phases were separated. The organic phase was washed with water (2x 50 mL) and brine (50 mL), dried over anhydrous sodium sulfate, filtered, and the solvent was evaporated. The crude product was purified by column chromatography (SiO₂, n-heptane/ethyl acetate 9:1), affording 2.84 g (9.5 mmol; 62%) of the desired product' as a red oil.

¹H NMR (CDCl₃, 400 MHz): δ = 0.91 (t, 3H; J = 7.3 Hz; CH₃), 1.09 (t, 3H; J = 7.4 Hz; CH₃), 1.12 (t, 3H; J = 7.4 Hz; CH₃), 1.31 - 1.45 (m, 2H; CH₂), 1.56 - 1.67 (m, 2H; CH₂), 2.02 - 2.31 (m, 4H; CH₂), 4.36 (t, 1H; *J* = 6.2 Hz; CH), 5.72 (s, 1H; =CH), 6.10 (s, 1H; =CH), 6.87 - 6.92 (m, 2H; ArH), 8.10 - 8.17 (m, 2H; ArH).

¹³C NMR (CDCl₃, 100.6 MHz): δ = 11.8 (CH₃), 13.8 (CH₃), 14.3 (CH₃), 19.3 (CH₂), 25.5 (CH₂), 27.4 (CH₂), 33.5 (CH₂), 58.2 (CH), 112.6 (=CH), 117.9 (=CH), 118.1 (=CH), 124.8 (=C), 125.8 (=CH), 137.9 (=C), 138.9 (=C), 150.8 (=C).

### Example 4

### Step 1: Synthesis of 1-(4-bromophenyl)-3,5-diethyl-2-propyl-1,2-dihydropyridine

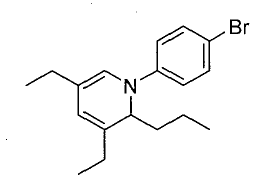

n-Butyraldehyde (49.25 g; 0.68 mol) was slowly added to a mixture of acetic acid (2.18 g, 36.3 mmol), 4-bromoaniline (25.00 g, 0.14 mol) in water (20 mL). The reaction mixture was heated to 45 °C for 1 h and to 75 °C for 20 h. Upon cooling to RT, n-heptane (100 mL) and water (100 mL) were added, and the phases were separated. The organic phase was washed with water (2x 100 mL) and brine (100 mL), dried over anhydrous sodium sulfate, filtered, and the solvent was evaporated. The crude product was purified by column chromatography (SiO₂, n-heptane), affording 31.07 g (92.9 mmol; 64%) of the desired product as a yellow liquid.

¹H NMR (CDCl₃, 400 MHz): δ = 0.89 (t, 3H; J = 7.3 Hz; CH₃), 1.04 (t, 3H; J = 7.4 Hz; CH₃), 1.09 (t, 3H; J = 7.4 Hz; CH₃), 1.32 - 1.44 (m, 2H; CH₂), 1.50 - 1.61 (m, 2H; CH₂), 1.95 - 2.27 (m, 4H; CH₂), 4.19 (t, 1H; *J* = 6.6 Hz; CH), 5.68 (s, 1H; =CH), 5.98 (s, 1H; =CH), 6.74 - 6.851 (m, 2H; ArH), 7.26 - 7.34 (m, 2H; ArH).

¹³C NMR (CDCl₃, 100.6 MHz): δ = 11.9 (CH₃), 14.3 (CH₃), 14.5 (CH₃), 19.4 (CH₂), 25.5 (CH₂), 27.6 (CH₂), 33.6 (CH₂), 58.7 (CH), 111.1 (=C), 116.2 (=CH), 118.1 (=CH), 119.5 (=CH), 121.0 (=C), 131.7 (=CH), 134.7 (=C), 145.3 (=C).

### Step 2: Synthesis of 4-(3,5-diethyl-2-propylpyridin-1(2H)-yl)benzonitrile DHP4

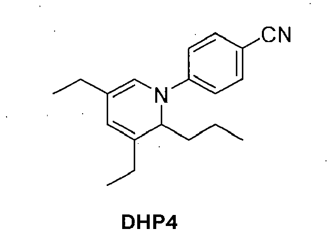

A flask was charged with 1-(4-bromophenyl)-3,5-diethyl-2-propyl-1,2-dihydropyridine (15.80 g, 47.3 mmol), potassium hexacyanoferrate(II) trihydrate (4.99 g, 11.8 mmol), palladium(II) acetate (0.16 g, 0.71 mmol), dicyclohexyl[2-(2,4,6-triisopropylphenyl)phenyl]phosphane (0.68 g, 1.42 mmol), and potassium carbonate (1.63 g, 11.8 mmol). The flask was evacuated and placed under argon. *N*,*N*-Dimethylformamide (50 mL) was added *via* syringe. The reaction mixture was degassed, purged with argon, and heated to 130 °C for 24 h. Upon cooling to room temperature, ethyl acetate (200 mL) and water (100 mL) were added, and the phases were separated. The organic phase was washed with water (2x 100 mL) and brine (100 mL), dried over anhydrous sodium sulfate, filtered, and the solvent was evaporated. The crude product was purified by column chromatography (SiO₂, n-heptane/ethyl acetate 9:1), affording 1.59 g (5.7 mmol; 12%) of the desired product as a yellow oil.

¹H NMR (CDCl₃, 400 MHz): δ = 0.90 (t, 3H; J = 7.3 Hz; CH₃), 1.07 (t, 3H; J = 7.4 Hz; CH₃), 1.11 (t, 3H; *J* = 7.4 Hz; CH₃), 1.31 - 1.45 (m, 2H; CH₂), 1.52 - 1.65 (m, 2H; CH₂), 2.00 - 2.29 (m, 4H; CH₂), 4.28 (t, 1H; J = 6.2 Hz; CH), 5.70 (s, 1H; =CH), 6.04 (s, 1H; =CH), 6.90 (d, 2H; *J* = 8.9 Hz; ArH), 7.49 (d, 2H; *J* = 8.9 Hz; ArH).

¹³C NMR (CDCl₃, 100.6 MHz): δ = 11.8 (CH₃), 13.9 (CH3), 14.4 (CH₃), 19.3 (CH₂), 25.5 (CH₂), 27.5 (CH₂), 33.4 (CH₂), 58.0 (CH), 100.3 (=C), 113.8 (=CH), 118.1 (=CH), 118.1 (=CH), 120.0 (=C), 123.6 (=C), 133.3 (=CH), 137.0 (=C), 149.0 (=C).

### Example 5

### Preparation of luting materials

The catalyst pastes C1-C9 listed in Table 1 were prepared by homogenous mixing the components named in the table. The pastes contain the following monomers: Bis-GMA, bis-[(2-methacryloyloxyethoxycarbonyl)amino]2,2,4(2,4,4)-trimethylhexane (UDMA), 2-(2-phenyl-phenoxy)ethyl methacrylate (PEM), V-380, 2-{[(2-(methacryloyloxy)ethoxy)carbonyl]amino} ethyl methacrylate (V-837), 1,10-decanediol dimethacrylate (D3MA). 10-(Methacryloyloxy)-decyl dihydrogen phosphate (MDP), 2-carboxyethyl acrylate (CEA) and ethyl 2-[4-(dihydroxyphosphoryl)-2-oxa-butyl]acrylate (EDOBA) were used as acidic monomers. Paste C7 contains no acidic monomer. Methylhydroquinone (MEHQ) was added as a stabilizer in each paste. Tertiary-amyl hydroperoxide (Luperox TAH), tert-butyl peroxybenzoate (Luperox P) and cumene hydroperoxide (CHP) were used as oxidants. Pyrogenic silica (HDK 2000, Wacker Chemie AG; BET surface area 120 m²/g), ytterbium fluoride (YbF₃; average particles size: 800 nm) and isofiller (a hot-cured composite filler ground to the desired grain size and prepared from UDMA (23.58 % by weight), 1,10-decanediol dimethacrylate (5.92 % by weight), dibenzoyl peroxide (0.50 % by weight), fumed silica Aerosil OX50 (50.0 % by weight) and YbF₃ (20.0 % by weight) were used as fillers.

**Table 1: Composition of Catalyst Pastes [wt.-%]**

| **Component** | **C1 (wt%)** | **C2 (wt%)** | **C3 (wt%)** | **C4* (wt%)** | **C5 (wt%)** | **C6 (wt%)** | **C7* (wt%)** | **C8 (wt%)** | **C9 (wt%)** |
|---|---|---|---|---|---|---|---|---|---|
| **Bis-GMA** | 7.418 | 6.982 | 7.418 | 8.353 | - | - | - | - | - |
| **UDMA** | 9.892 | 9.309 | 9.892 | 11.139 | - | - | - | - | - |
| **PEM** | 7.418 | 6.982 | 7.418 | 8.353 | - | - | - | - | - |
| **V380** | - | - | - | - | 11.055 | 10.764 | 11.347 | 9.31 | 9.31 |
| **V837** | - | - | - | - | 11.056 | 10.764 | 11.347 | 9.31 | 9.31 |
| **D3MA** | - | - | - | - | 5.528 | 5.383 | 5.673 | 4.655 | 4.655 |
| **MDP** | 2.911 | - | - | - | 0.728 | 1.456 | - | 4.365 | 4.365 |
| **CEA** | - | 4.366 | - | - | - | - | - | - | - |
| **EDOBA** | - | - | 2.911 | - | - | - | - | - | - |
| **LuperoxTAH** | 1.456 | 1.456 | 1.456 | - | - | - | - | - | - |
| **Luperox** P | - | - | - | - | 0.728 | 0.728 | 0.728 | 1.455 | - |
| **CHP** | - | - | - | 1.250 | - | - | - | - | 1.455 |
| **MEHQ** | 0.015 | 0.015 | 0.015 | 0.015 | 0.015 | 0.015 | 0.015 | 0.015 | 0.015 |
| **HDK 2000** | 3.300 | 3.300 | 3.300 | 3.300 | 3.300 | 3.300 | 3.300 | 3.300 | 3.300 |
| **YbF₃** | 43.900 | 43.900 | 43.900 | 43.900 | 43.900 | 43.900 | 43.900 | 43.900 | 43.900 |
| **Isofiller** | 23.690 | 23.690 | 23.690 | 23.690 | 23.690 | 23.690 | 23.690 | 23.690 | 23.690 |

| | | | | | | | | | |
|---|---|---|---|---|---|---|---|---|---|
| * comparative example | | | | | | | | | |

The base pastes B1-B6 listed in Table 2 were prepared by homogeneous mixing of the components named in the table. TEMPO (CAS Registry Number 2564-83-2) was used as additional stabilizer. The barium-aluminium-borosilicate glass filler GM27884 (Schott AG, average particle size 1 µm; BET specific surface area (BET DIN ISO 9277: 2010) 3.9 m²/g; composition (% by weight ): Al₂O₃: 10, B₂O₃: 10, BaO: 25 and SiO₂: 55) was used. For the reference paste, acetylthiourea (ATU) was used as a reducing agent.

**Table 2: Composition of Base Pastes [wt.-%]**

| **Component** | **B1 (wt%)** | **B2 (wt%)** | **B3 (wt%)*** | **B4 (wt%)** | **B5 (wt%)** | **B6 (wt%)** |
|---|---|---|---|---|---|---|
| **Bis-GMA** | 10.277 | 10.256 | 10.330 | - | - | - |
| **UDMA** | 13.703 | 13.675 | 13.775 | - | - | - |
| **PEM** | 10.277 | 10.256 | 10.330 | - | - | - |
| **V380** | - | - | - | 13.704 | 13.703 | 13.679 |
| **V837** | - | - | - | 13.704 | 13.703 | 13.679 |
| **D3MA** | - | - | - | 6.852 | 6.851 | 6.840 |
| **DHP1** | 0.700 | - | - | 0.700 | 0.700 | - |
| **DHP2** | - | 0.770 | - | - | - | - |
| **DHP4** | - | - | - | - | - | 0.770 |
| **ATU** | - | - | 0.525 | - | - | - |
| **Cu(acac)₂** | 0.007 | 0.007 | 0.004 | 0.004 | 0.007 | 0.007 |
| **MEHQ** | 0.018 | 0.018 | 0.018 | 0.018 | 0.018 | 0.018 |
| **TEMPO** | 0.018 | 0.018 | 0.018 | 0.018 | 0.018 | 0.007 |
| **Glass filler** | 65.00 | 65.00 | 65.00 | 65.00 | 65.00 | 65.00 |

| | | | | | | |
|---|---|---|---|---|---|---|
| * comparative example | | | | | | |

### Example 6

### Measurement of the mechanical properties (flexural strength and modulus)

The cements listed in Table 3 were prepared by mixing catalyst paste and base paste by hand in a ratio of 1/1 wt/wt. Flexural strength specimens (2 mm * 2 mm * 25 mm) were prepared using a stainless-steel mold. The mold was filled with the cement, then covered with a polyethylene film and placed in an oven at 37 °C for 45 min. The specimens were removed from the mold and were stored in water at 37 °C for 24 h. The measurement of the flexural strength and modulus was carried out in three-point bending tests (span: 20 mm) according to IS04049 (Dentistry - Polymer-based filling, restorative and luting materials) with a speed of 0.8 mm min⁻¹ using a Z2.5/TS universal testing machine (Zwick, Germany).

**Table 3: Mechanical Properties of Cements**

| **Cement** | **Flexural strength [MPa]** | **Flexural modulus [GPa]** |
|---|---|---|
| **C4 + B3*** | 89.6 ± 8.5 | 6.4 ± 0.1 |
| **C7 + B4*** | 54.0 ± 6.1 | 2.5 ± 0.2 |
| **C7 + B5*** | 55.3 ± 2.6 | 2.7 ± 0.2 |
| **C5 + B4** | 85.1 ± 5.0 | 5.1 ± 0.1 |
| **C6 + B4** | 91.5 ± 6.9 | 6.1 ± 0.2 |
| **C1 + B1** | 78.9 ± 9.9 | 5.0 ± 0.2 |
| **C1 + B2** | 84.5 ± 7.5 | 5.9 ± 0.2 |
| **C2 + B1** | 88.8 ± 5.4 | 5.5 ± 0.2 |
| **C3 + B1** | 79.7 ± 7.9 | 6.3 ± 0.3 |
| **C8 + B6** | 79.5 ± 6.8 | 5.0 ± 0.1 |
| **C9 + B6** | 84.3 ± 5.5 | 5.1 ± 0.1 |

| | | |
|---|---|---|
| * comparative example | | |

The compositions according to the invention had similar mechanical properties than the reference material comprising a hydroperoxide (CHP) and the thiourea derivative ATU (C4+B3). However, they have no bitter taste. The results also show that compositions comprising a peroxide and a dihydropyridine derivative but no acidic monomer (C7+B4 and C7+B5) have worse mechanical properties than the reference material. The addition of an acidic monomer to the compositions comprising peroxide and a dihydropyridine derivation leads to a significant improvement of the mechanical properties (C5+B4; C6+B4).. The mechanical properties of cement C6+B4 comprising a higher amount of the acidic monomer MDP are better than those of cement C5+B4 with a lower amount of acidic monomer. The results also show that similar results can be achieved with other combinations of acidic monomers and dihydropyridine derivatives.

### Example 7

### Measurement of the working time

The cements listed in Table 4 were prepared by mixing catalyst paste and base paste by hand in a ratio of 1/1 wt/wt. The working time was determined with an oscillating rheometer (Rheometer MCR 302, Anton Paar) using a plate / plate geometry, at 28.7 °C. The storage modulus was recorded as a function of time (frequency = 1 Hz). A graph representing the storage modulus (logarithmic) as a function of time (non-logarithmic) was then used for the determination of the working time. The inflexion point (to be found where the slope of the function reaches its highest value) was identified. A second point on the curve was set as soon as the storage modulus started to rise (end of the stable phase). A straight line was then drawn between the two points. The point on the curve (before the inflexion point) where the tangent line is parallel to this straight line gives the working time.

**Table 4: Working times of Cements**

| **Cement** | **Working time [s]** |
|---|---|
| **C4 + B3*** | 288 ± 10 |
| **C7 + B4*** | Slow settinq (not measurable) |
| **C7 + B5*** | Slow settinq (not measurable) |
| **C5 + B4** | 221 ± 14 |
| **C6 + B4** | 82 ± 7 |
| **C1 + B1** | 171 ± 2 |
| **C1 + B2** | 72 ± 11 |
| **C2 + B1** | 157 ± 2 |
| **C3 + B1** | 250 ± 6 |
| **C8 + B6** | 102 ± 5 |
| **C9 + B6** | 125 ± 5 |

| | |
|---|---|
| * comparative example | |

The cements according to the present invention polymerized faster than the reference material containing the thiourea derivative ATU. A comparison of the setting times of cements C7+B4 and C7+B5 with the results of cements C5+B4 and C6+B4 shows that the presence of the acidic monomer significantly accelerates curing of the material.

## Claims

1. A dental material which comprises a catalyst paste and a base paste, wherein the catalyst paste comprises:
(a) at least one polyfunctional radically polymerizable monomer without an acid group,
(b) optionally at least one monofunctional radically polymerizable monomer without an acid group,
(c) at least a radically polymerizable monomer and/or oligomer with an acid group
(d) at least one oxidizing agent; and
wherein the base paste comprises:
(e) at least one polyfunctional radically polymerizable monomer without an acid group,
(f) optionally at least one a monofunctional radically polymerizable monomer without an acid group, and
(g) at least one dihydropyridine derivative according to Formula (I) 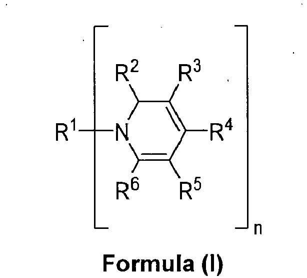 in which
R¹ is a C₆-C₁₀ aryl group, preferably a phenyl group, which can be unsubstituted or substituted by one or more substituents which are selected from branched or preferably linear C₁-C₂₀ alkyl groups, preferably C₁-C₁₂ alkyl groups, and more preferably C₁-C₆ alkyl groups, C₆-C₁₀ aryl groups, -O-C₁-C₁₂ alkoxy groups, -S-C₁-C₁₂ sulfide groups, CN, NO₂, ester groups -C(O)O-R⁷, where R⁷ is either a branched or preferably linear C₁-C₂₀ alkyl group, a C₆-C₁₀ aryl group or a C₇-C₂₀-alkylaryl group, and/or acyl groups -C(O)-R⁸, where R⁸ is either a branched or preferably linear C₁-C₂₀ alkyl group, a C₆-C₁₀ aryl group or a C₇-C₂₀-alkylaryl group;
R²⁻⁶ independently of each other are a hydrogen atom; a branched or preferably linear C₁-C₂₀ alkyl group., preferably a C₁-C₁₂ alkyl group, and more preferably a C₁-C₆ alkyl group, that can be interrupted by one or more S atoms, O atoms, -C(O)O- groups and/or -C(O)NH- groups, and that can be unsubstituted or substituted by one or more substituents selected from C₁-C₂₀ alkyl groups, -O-C₁-C₁₂ alkoxy groups and/or ester groups -C(O)O-R⁷, where R⁷ is either a branched or preferably linear C₁-C₂₀ alkyl group, a C₆-C₁₀ aryl group or a C₇-C₂₀-alkylaryl group; a C₃-C₁₆ cycloaliphatic group that can be unsubstituted or substituted by one or more substituents selected from branched or preferably linear C₁-C₂₀ alkyl groups, preferably C₁-C₁₂ alkyl groups, most preferably C₁-C₆ alkyl groups, and that can be interrupted by one or more S atoms, O atoms, -C(O)O- groups and/or -C(O)NH- groups ; or a C₇-C₂₀-alkylaryl group that can be interrupted by one or more S atoms, O atoms, -C(O)O- groups and/or -C(O)NH- groups, and/or can be unsubstituted or substituted by one or more substituents selected from branched or preferably linear C₁-C₂₀ alkyl groups, C₆-C₁₀ aryl groups, branched or preferably linear -O-C₁-C₁₂ alkoxy groups, ester groups -C(O)O-R⁷, where R⁷ is either a branched or preferably linear C₁-C₂₀ alkyl group, a C₆-C₁₀ aryl group or a C₇-C₂₀-alkylaryl group, and/or acyl groups -C(O)-R⁸, where R⁸ is either a branched or preferably linear C₁-C₂₀ alkyl group, a C₆-C₁₀ aryl group or a C₇-C₂₀-alkylaryl group;
n is 1, 2, 3 or 4, preferably 1, 2 or 3, more preferably 1 or 2,
and wherein the material comprises at least 50 % by weight of polyfunctional radically polymerizable monomers or monomers and oligomers, based on the total amount of radically polymerizable monomers and oligomers.

2. The composition according to claim 1, wherein the variables of Formula (I) have the following meanings:
R¹ is a C₆-C₁₀ aryl group, preferably a phenyl group, which can be unsubstituted or substituted by one or more, preferably 1 to 3, more preferably 1 or 2, and most preferably 1 substituent which is/are preferably selected from C₆-C₁₀ aryl groups, -O-C₁-C₁₂ alkoxy groups, -O-C₁-C₁₂ sulfide groups, CN, NO₂, ester groups -C(O)O-R⁷, where R⁷ is a branched or preferably linear C₁-C₂₀ alkyl group, and/or acyl groups -C(O)-R⁸, where R⁸ is a branched or preferably linear C₁-C₂₀ alkyl group;
R⁴, R⁶ are each a hydrogen atom;
R², R³, R⁵ independently of each other are each a branched or preferably linear C₁-C₂₀, preferably C₁-C₁₆, more preferably C₁-C₁₀, and most preferably C₁-C₆ alkyl group that can be interrupted by one or more S atoms, O atoms, -C(O)O-groups and/or -C(O)NH- groups and is preferably not interrupted, a C₃-C₁₆ cycloaliphatic group that is preferably unsubstituted or is substituted by one or more, preferably 1 to 3, more preferably 1 or 2, and most preferably 1 branched or preferably linear C₁-C₁₀ alkyl group that can be interrupted by one or more, preferably 1 to 3, more preferably 1 or 2 and even more preferably 1 S atom, O atom, -C(O)O- group and/or -C(O)NH- group and is most preferably not interrupted; or a C₇-C₂₀-alkylaryl group that can be interrupted by one or more, preferably 1 to 3, more preferably 1 or 2, and even more preferably 1 S atom, O atom, -C(O)O- group and/or -C(O)NH- group and is most preferably not interrupted, and/or can be substituted by one or more, preferably 1 to 3, more preferably 1 or 2, and even more preferably 1 substituent selected from branched or preferably linear C₁-C₂₀ alkyl groups, C₆-C₁₀ aryl groups, -O-C₁-C₁₂ alkoxy groups, ester groups -C(O)O-R⁷, where R⁷ is either a branched or preferably linear C₁-C₂₀ alkyl group, a C₆-C₁₀ aryl group or a C₇-C₂₀-alkylaryl group, and/or acyl groups -C(O)-R⁸, where R⁸ is either a branched or preferably linear C₁-C₂₀ alkyl group, a C₆-C₁₀ aryl group or a C₇-C₂₀-alkylaryl group, and is most preferably not substituted,
n 1.

3. The composition according to claim 1, wherein
R¹ is a phenyl group, which is unsubstituted or substituted by one substituent selected from a phenyl group, linear -O-C₁-C₄ alkoxy groups, preferably -OCH₃, -O-C₁-C₄ sulfide groups, preferably -SCH₃, CN, NO₂, ester groups -C(O)O-R⁷, where R⁷ is a branched or preferably linear C₁-C₄ alkyl group, preferably methyl or ethyl;
R⁴, R⁶ are each a hydrogen atom;
R², R³, R⁵ independently of each other are each H, preferably a C₇-C₁₀-alkylaryl group, especially -CH₂-Ph or -CH₂CH₂-Ph, more preferably a linear C₁-C₆ alkyl group that can be substituted by one -C(O)O-R⁷ group, where R⁷ is ethyl or preferably methyl, or is preferably unsubstituted,
n 1,
and wherein the residues R² to R⁶ have the meanings defined in claim 1 or 2.

4. The composition according to any one of claims 1 to 3, wherein the at least one oxidizing agent is a peroxide, a hydroperoxide, or a mixture thereof.

5. The composition according to any one of the preceding claims, wherein the at least one polyfunctional monomer (a) and the at least one polyfunctional monomer (e) are selected from a polyfunctional (meth)acrylate, preferably from bisphenol-A-dimethacrylate, bis-GMA (an addition product of methacrylic acid and bisphenol-A-diglycidyl ether), ethoxy- or propoxylated bisphenol-A-dimethacrylates, such as the bisphenol A dimethacrylate SR-348c (Sartomer) with 3 ethoxy groups or 2,2-bis[4-(2-methacryloyloxypropoxy)phenyl]propane, urethanes of 2-(hydroxymethyl)-acrylic acid and diisocyanates, such as 2,2,4-trimethylhexamethylene diisocyanate or isophoronic diisocyanate, UDMA (an addition product of 2-hydroxyethyl methacrylate and 2,2,4-trimethylhexamethylene-1,6-diisocyanate), 2-{[(2-(methacryloyloxy)ethoxy)carbonyl]-amino} ethyl methacrylate (V-837), tetramethylxylylene diurethane ethylene glycol di(meth)acrylate and tetramethylxylylene diurethane ethylene glycol di(meth)acrylate, respectively, V380 having the following formula 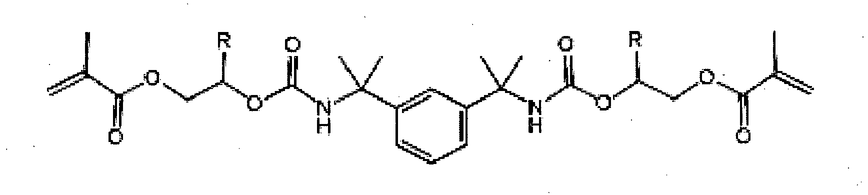 wherein the R groups are each independently H or CH₃, di-, tri- or tetraethylene glycol dimethacrylate, trimethylol propanetrimethacrylate, pentaerythritol tetramethacrylate and glycerol di- and trimethacrylate, 1,4-butanediol dimethacrylate, 1,10-decanediol dimethacrylate (D3MA), bis(methacryloyloxymethyl)tricyclo-[5.2.1.02,6]decane (DCP), polyethylene glycol or polypropylene glycol dimethacrylates, such as polyethylene glycol 200-dimethacrylate or polyethylene glycol 400-dimeth¬acrylate (PEG-200-DMA or PEG-400-DMA) or 1,12-dodecanediol dimethacrylate, and a mixture thereof, preferably Bis-GMA, UDMA, V-380, triethylene glycol dimethacrylate (TEGDMA), PEG-400-DMA (NK ester 9G) or a mixture thereof.

6. The composition according to any one of the preceding claims, wherein the monofunctional monomers (b) and (f) are selected from monofunctional (meth)acrylates, preferably from benzyl, tetrahydrofurfuryl, isobornyl (meth)acrylate, p-cumylphenoxyethylene glycol methacrylate (CMP-1E), 2-([1,1'-biphenyl]-2-oxy)ethyl methacrylate (PEM = MA-836), tricyclodecane methyl (meth)acrylate, 2-(2-biphenyloxy)ethyl(meth)acrylate and a mixture thereof.

7. The composition according to any one of the preceding claims, wherein the at least one radically polymerizable monomer and/or oligomer with an acid group comprises at least one acid group selected from a carboxylic acid group, phosphoric acid ester group and phosphonic acid group, preferably a phosphonic acid group or phosphoric acid ester group, more preferably a monohydrogen phosphate or dihydrogen phosphate group.

8. The composition according to claim 7, wherein the at least one radically polymerizable monomer comprising an acid group is selected from
monomers comprising a carboxylic acid group, preferably from 4-(meth)acryloyloxyethyltrimellitic acid, 10-methacryloyloxydecylmalonic acid, N-(2-hydroxy-3-methacryloyloxypropyl)-N-phenylglycine and 4-vinylbenzoic acid and 2-carboxyethyl acrylate (CEA),
monomers comprising a phosphonic acid group, preferably 4-vinylbenzylphosphonic acid, 2-[4-(dihydroxyphosphoryl)-2-oxa-butyl]-acrylic acid and its esters, such as its ethyl ester, 2-[4-(dihydroxyphosphoryl)-2-oxa-butyl]acrylate (EDOBA) and 2,4,6-trimethylphenyl 2-[4-(dihydroxyphosphoryl)-2-oxa-butyl]acrylate,
monomers comprising a monohydrogen phosphate group, preferably bis[(2-methacryloyloxy)ethyl] phosphate and bis[(6-methacryloyloxy)hexyl] phosphate,
monomers comprising a dihydrogen phosphate group, preferably 2-methacryloyloxyethylphenyl hydrogen phosphate, 10-methacryloyloxydecyl dihydrogen phosphate (MDP), glycerine dimethacrylate dihydrogen phosphate, and dipentaerythritol pentamethacryloyloxy phosphate,
and mixtures thereof.

9. The composition according to any of the preceding claims, wherein the base paste additionally comprises at least one transition metal compound, that is preferably selected form the compounds of copper, cobalt, nickel, vanadium, platinum, palladium, manganese, or a mixture thereof, preferably copper, most preferably copper(II) acetylacetonate.

10. The composition according to any one of the preceding claims, which additionally comprises at least one photoinitiator for the radical polymerization.

11. The composition according to any one of the preceding claims, which additionally comprises at least one filler, preferably a radiopaque glass filler, a composite filler, or a mixture thereof.

12. The composition according to any one of the preceding claims,
wherein the catalyst paste comprises
(a) 4 to 78 % by weight, preferably 10 to 58 % by weight, more preferably 15 to 49 % by weight, of at least one radically polymerizable monomer without an acid group,
(b) 0 to 40 % by weight, preferably 0 to 30 % by weight, more preferably 0 to 25 % by weight, of at least one monofunctional radically polymerizable monomer without an acid group,
(c) 0.1 to 40 % by weight, preferably 0.3 to 15 % by weight, more preferably 0.5 to 10 % by weight, of at least one radically polymerizable monomer and/or oligomer, preferably a monomer, comprising an acid group,
(d) 0.05 to 8 % by weight, preferably 0.25 to 5 % by weight, more preferably 0.5 to 3 % by weight, of at least one peroxide and/or hydroperoxide,
(i) 0 to 2 % by weight, preferably 0 to 1.5 % by weight, more preferably 0 to 1.0 % by weight, of one or more photoinitiators,
(j) 20 to 90 % by weight, preferably 40 to 80 % by weight, more preferably 50 to 80 % by weight, of at least one filler, and
(k) 0 to 2 % by weight preferably 0 to 1 % by weight, more preferably 0 to 1 % by weight, of one or more additives,
based on the total mass of the catalyst paste,
and wherein the base paste comprises
(e) 9 to 79 % by weight, preferably 19 to 60 % by weight, more preferably 19 to 49 % by weight, of at least one radically polymerizable monomer without an acid group,
(f) 0 to 40 % by weight, preferably 0 to 30 % by weight, more preferably 0 to 25 % by weight, of at least one monofunctional radically polymerizable monomer without an acid group,
(g) 0.05 to 5% by weight, preferably 0.3 to 3 % by weight, more preferably 0.3 to 2 % by weight, of at least one dihydropyridine derivative according to Formula (I),
(h) 1 to 500 ppm, preferably 10 to 200 ppm, more preferably 10 to 100 ppm, of at least one transition metal compound,
(i) 0 to 2 % by weight, preferably 0 to 1.5 % by weight, more preferably 0 to 1 % by weight, of one or more photoinitiators,
(j) 20 to 90 % by weight, preferably 40 to 80 % by weight, more preferably 50 to 80 % by weight, of at least one filler, and
(k) 0 to 2 % by weight, preferably 0 to 1 % by weight, more preferably 0 to 1 % by weight, of one or more additives,
based on the total mass of the base paste, and wherein the material comprises at least 50 % by weight, preferably at least 60 % by weight, and more preferably at least 70 % by weight, of polyfunctional radically polymerizable monomers or monomers and oligomers, based on the total amount of radically polymerizable monomers and oligomers.

13. Composition according to any of the preceding claims for therapeutic use as a dental material, preferably as dental cement, layering or veneering material, restorative composite or luting cement.

14. Non-therapeutic use of a composition according to any one of claims 1 to 12 for the manufacture or repair of dental restorations, in particular inlays, onlays, crowns or bridges.

15. A dihydropyridine derivative according to Formula (I) 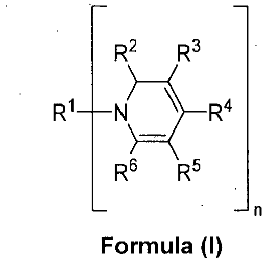 in which
R¹ is a C₆-C₁₀ aryl group, preferably a phenyl group, which is substituted by one or more substituents, which are preferably selected from branched or preferably linear C₁-C₂₀ alkyl groups, C₆-C₁₀ aryl groups, -O-C₁-C₁₂ alkoxy groups, -S-C₁-C₁₂ sulfide groups, CN, NO₂, ester groups -C(O)O-R⁷, where R⁷ is either a branched or preferably linear C₁-C₂₀ alkyl group, a C₆-C₁₀ aryl group or a C₇-C₂₀-alkylaryl group, and/or acyl groups -C(O)-R⁸, where R⁸ is either a branched or preferably linear C₁-C₂₀ alkyl group, a C₆-C₁₀ aryl group or a C₇-C₂₀-alkylaryl group,
R²⁻⁶ independently of each other are a hydrogen atom; a branched or preferably linear C₁-C₂₀ alkyl group, preferably a C₁-C₁₂ alkyl group, and more preferably a C₁-C₆ alkyl group, that can be interrupted by one or more S atoms, O atoms, -C(O)O- groups and/or -C(O)NH- groups, and that can be unsubstituted or substituted by one or more substituents selected from C₁-C₂₀ alkyl groups, -O-C₁-C₁₂ alkoxy groups and/or ester groups -C(O)O-R⁷, where R⁷ is either a branched or preferably linear C₁-C₂₀ alkyl group, a C₆-C₁₀ aryl group or a C₇-C₂₀-alkylaryl group; a C₃-C₁₆ cycloaliphatic group that can be unsubstituted or substituted by one or more substituents selected from branched or preferably linear C₁-C₂₀ alkyl groups, preferably C₁-C₁₂ alkyl groups, most preferably C₁-C₆ alkyl groups, and that can be interrupted by one or more S atoms, O atoms, -C(O)O- groups and/or -C(O)NH- groups ; or a C₇-C₂₀-alkylaryl group that can be interrupted by one or more S atoms, O atoms, -C(O)O- groups and/or -C(O)NH- groups, and/or can be unsubstituted or substituted by one or more substituents selected from branched or preferably linear C₁-C₂₀ alkyl groups, C₆-C₁₀ aryl groups, branched or preferably linear -O-C₁-C₁₂ alkoxy groups, ester groups -C(O)O-R⁷, where R⁷ is either a branched or preferably linear C₁-C₂₀ alkyl group, a C₆-C₁₀ aryl group or a C₇-C₂₀-alkylaryl group, and/or acyl groups -C(O)-R⁸, where R⁸ is either a branched or preferably linear C₁-C₂₀ alkyl group, a C₆-C₁₀ aryl group or a C₇-C₂₀-alkylaryl group;
n is 1, 2, 3 or 4, preferably 1, 2 or 3, more preferably 1 or 2.
